# EUROPEAN PATENT SPECIFICATION

(11) **EP 4 277 533 B1**
(45) Date of publication and mention of the grant of the patent: **16.09.2026**
(21) Application number: 22703184.6
(22) Date of filing: 17.01.2022
(51) Int. Cl.: A61B 8/08

(54) **SYSTEM AND METHOD FOR NON-INVASIVE DETERMINATION OF BLADDER OVERACTIVITY USING ULTRASOUND VIBROMETRY**
SYSTEM UND VERFAHREN ZUR NICHT-INVASIVEN BESTIMMUNG DER BLASENÜBERAKTIVITÄT UNTER VERWENDUNG VON ULTRASCHALLVIBROMETRIE
SYSTÈME ET PROCÉDÉ DE DETERMINATION NON INVASIVE D'UNE SURACTIVITE DE LA VESSIE PAR VIBROMETRIE A ULTRASONS

(30) Priority: 15.01.2021 US 202163138116 P
(43) Date of publication of application: 22.11.2023
(73) Proprietor: Mayo Foundation for Medical Education and Research, Rochester, Minnesota 55905 (US)
(72) Inventor: FATEMI, Mostafa, Rochester, Minnesota 55902-4507 (US); ALIZAD, Azra, Rochester, Minnesota 55902-4507 (US); ROSEN, David P., Rochester, Minnesota 55901 (US)
(74) Representative: Samson & Partner Patentanwälte mbB
(86) International application number: PCT/US2022/012675
(87) International publication number: WO 2022/155573

(56) References cited:
- US-A1- 2014 296 709
- IVAN Z NENADIC ET AL: "Lamb wave dispersion ultrasound vibrometry (LDUV) method for quantifying mechanical properties of viscoelastic solids;LDUV method for quantifying mechanical properties of viscoelastic solids", PHYSICS IN MEDICINE AND BIOLOGY, INSTITUTE OF PHYSICS PUBLISHING, BRISTOL GB, vol. 56, no. 7, 14 March 2011 (2011-03-14), pages 2245 - 2264, XP020188434, ISSN: 0031-9155, DOI: 10.1088/0031-9155/56/7/021
- H. KANAI: "Propagation of spontaneously actuated pulsive vibration in human heart wall and in vivo viscoelasticity estimation", IEEE TRANSACTIONS ON ULTRASONICS, FERROELECTRICS AND FREQUENCY CONTROL, vol. 52, no. 11, 1 November 2005 (2005-11-01), pages 1931 - 1942, XP055006428, ISSN: 0885-3010, DOI: 10.1109/TUFFC.2005.1561662

## Description

### STATEMENT REGARDING FEDERALLY SPONSORED RESEARCH

This invention was made with government support under DK099231 awarded by the National Institutes of Health. The government has certain rights in the invention.

### BACKGROUND

The present disclosure relates to systems and methods for analyzing bladder overactivity. More particularly, the present disclosure relates to a method for determining bladder activity, using ultrasound vibrometry to excite mechanical waves in the bladder wall and track the motion using ultrasound pulse-echo techniques.

Reduced bladder compliance (defined as changes in bladder volume due to a change in the detrusor muscle pressure) is of particular importance in subpopulations of patients with neurogenic bladder disorders who should be carefully monitored with urodynamic studies (UDS) on an annual basis to ensure that bladder pressures stay within normal parameters. Urodynamic studies are currently considered the gold standard in clinical assessment for measuring bladder compliance but are uncomfortable and carry risks of infection. A typical procedure lasts approximately 45 minutes, requires catheter placement in the bladder and the vagina or rectum, filling the bladder at a defined rate, and measuring the change in detrusor pressure (Pdet) defined as detrusor pressure as the bladder fills. A compliant (elastic) bladder expands to accommodate the filling volume, resulting in a low detrusor pressure, while a non-compliant (inelastic) bladder does not expand as readily, and the detrusor pressure rises during bladder filling. The urodynamic studies are often accompanied by pain during and following the procedure, traumas and infection due to urinary catheterization have been reported in both men and women. In addition to discomfort and clinical risks, urodynamic studies are labor and resource intensive for the institutions that treat and follow these patients.

Detrusor overactivity (DO) is the urodynamic observation of involuntary contractions of the detrusor muscle of the bladder during the filling phase of micturition. These involuntary contractions produce fluctuations in the Pdet measured by UDS. DO is common in neurogenic bladder patients and is important to diagnose in this population due to the risk of upper urinary tract issues. In both neurogenic and non-neurogenic bladder patient populations, DO is also frequently accompanied by overactive bladder syndrome (OAB), which is characterized by urinary urgency as well as frequency and nocturia. OAB is believed to occur in 10% of the population and is associated with substantial reductions in quality-of-life.

Although assessment of DO in patients with lower urinary tract symptoms provides useful information for clinical decision making, it is generally only assessed when there is a substantial clinical need. For instance, it is generally recommended that DO only be assessed after conservative treatments have failed at treating non-neurogenic OAB. This is because of the potential for discomfort and morbidity associated with UDS, due to the catheterization of the urinary tract that is conventionally required for measuring bladder pressure. For instance, a review that considered the results reported for 963 non-neurogenic patients presented a rate of urinary tract infection after UDS of 28%.

Approaches to using ultrasound for assessment of DO have primarily focused on measuring geometric characteristics of the bladder. For instance, ultrasound has been used to measure bladder wall thickness, which has been proposed to correlate with DO due to detrusor hypertrophy. Likewise, changes in bladder shape during contraction have been measured through ultrasound imaging. Such approaches rely on the correlation between bladder shape and bladder mechanics. These and other available techniques also do not provide a non-invasive and relatively pain-free, quantitative measurement of DO. Instead, in clinical practice, clinicians often rely on urodynamic studies despite the above-described risks and drawbacks.

Therefore, there is a need for systems and methods to non-invasively and painlessly provide salient information regarding the properties of the bladder wall in patients who experience bladder dysfunction.

The document US 2014/296709 A1 discloses a system and a method for non-invasively determining viscoelasticity of the bladder by fitting an analytical Lamb wave model to dispersion data.

### SUMMARY

The present invention is set out in the appended set of claims. Disclosed herein is a system and method that is relatively pain-free and non-invasively determines viscoelasticity of the bladder, using ultrasound bladder vibrometry (UBV) to determine detrusor overactivity (DO). The present disclosure further overcomes the aforementioned drawbacks by providing a system and method that can be used for early evaluation and more frequent follow-ups of patients who experience incontinence. UBV is a non-invasive technique that uses ultrasound radiation force that is focused to excite Lamb waves at the wall and pulse-echo techniques to track the tissue deformation. In this regard, the term "bladder" may not refer to the "urinary" bladder in the human body, but may more generally refer to a tissue volume formed by tissue walls that may have some elasticity. Accordingly, the urinary bladder is one example of a bladder. Cross-spectral analysis can be used to calculate the wave velocity, which is related to the elastic properties of the bladder wall.

Distinct from a strictly shape-based assessment of the bladder, UBV is an elastographic technique that provides for more direct means of characterizing bladder elasticity and loading. Elastographic techniques have sensitivity to the changes in tissue elasticity produced by tissue loading, including those produced by large deformations and active muscle contraction. This sensitivity may be leveraged as a potential means of assessing and tracking pressures sustained by tissue in a variety of biological vessels, including the ventricles of the heart, the diaphragm muscle and the lungs, and large blood vessels. For the bladder, UBV has shown medium-to-strong correlations of UBV measured group velocity squared (GV2), and elasticity with UDS-determined Pdet.

Because UBV is sensitive to the elasticity and loading state of the bladder wall, the temporal fluctuations in detrusor pressure may be usefully detected by UBV measurement. In some configurations, DO may be detected by analysis of transient peaks in time-resolved series of UBV measurements. In a non-limiting example, a cohort of neurogenic bladder patients undergoing routine UDS were assessed for DO measurements. The timestamped GV2 data series were decomposed into an ensemble of transient peaks (ETP) and lower envelope (LE) data series. A DO index, *I*, was used for the characterization of these DO associated peaks. The same analysis was applied to the concurrently measured Pdet data series for comparison.

In accordance with one aspect described in the present disclosure, a system and method for non-invasively determining DO using ultrasound vibrometry is disclosed. The system uses ultrasound radiation force to "tap" the wall of the bladder and excite Lamb waves. The system further includes using pulse-echo ultrasound to track the motion of the wall. Fourier space analysis of the tissue motion is used to obtain the wave velocity dispersion (change of wave velocity as a function of frequency). An analytical Lamb wave model is fit to the dispersion data to estimate viscoelasticity of the elastic tissue volume. A parameter representing a property of a tissue volume, such as a detrusor overactivity, may then be determined for a subject.

In one aspect, a method is provided for characterizing a parameter representing a property of a tissue volume. The method includes, with a transducer, detecting ultrasonic energy reflected by multiple locations along the tissue volume that is subject to stress to form ultrasonic echo data. The tissue volume is formed by a tissue wall that spatially separates a fluid material from a rigid material. The method also includes generating an ensemble of transient peaks from the ultrasonic echo data, and determining the parameter representing a property of the tissue volume from the ensemble of transient peaks.

In some aspects of the method, the parameter representing a property of the tissue volume is at least one of group velocity (GV), group velocity squared (GV2), a power of group velocity, arrival time of an induced Lamb wave, estimated elastic properties of the tissue volume, detrusor pressure (Pdet), or a detrusor overactivity index of the tissue volume.

In one aspect, a system is provided for characterizing a parameter representing a property of a tissue volume. The system includes a transducer configured for detecting ultrasonic energy reflected by multiple locations along the tissue volume that is subject to stress to form ultrasonic echo data. The tissue volume is formed by a tissue wall that spatially separates a fluid material from a rigid material. The system also includes a processor configured to generate an ensemble of transient peaks from the ultrasonic echo data and determine the parameter representing a property of the tissue volume from the ensemble of transient peaks.

In some aspects of the system, the parameter representing a property of the tissue volume is at least one of group velocity (GV), group velocity squared (GV2), a power of group velocity, arrival time of an induced Lamb wave, estimated elastic properties of the tissue volume, detrusor pressure (Pdet), or a detrusor overactivity index of the tissue volume.

The foregoing and other aspects and advantages of the invention will appear from the following description. In the description, reference is made to the accompanying drawings that form a part hereof, and in which there is shown by way of illustration a preferred embodiment of the invention. Such embodiment does not necessarily represent the full scope of the invention, however, and reference is made therefore to the claims and herein for interpreting the scope of the invention.

### BRIEF DESCRIPTION OF THE DRAWINGS

FIG. 1 is a block diagram of an ultrasound radiation force system.
FIG. 2 is a block diagram of an ultrasound imaging system used in the system of FIG. 1.
FIG. 3 is a block diagram of a transmitter that forms part of the ultrasound imaging system of FIG. 2.
FIG. 4 is a block diagram of a receiver that forms part of the ultrasound imaging system of FIG. 2.
FIG. 5A is a flow chart of the steps using the ultrasound imaging system of FIG. 2 to measure detrusor overactivity.
FIG. 5B is another flow chart of a non-limiting example method for determining detrusor overactivity.
FIG. 5C is yet another flowchart of a non-limiting example method for determining detrusor overactivity.
FIG. 5D is a flow chart of the steps using the ultrasound imaging system of FIG. 2 to measure one or more properties of tissue.
FIG. 6A is a diagram of one example of the basic principle of ultrasound bladder vibrometry (UBV) described in the present disclosure.
FIG. 6B illustrates *in vivo* human B-mode of the bladder with a sketch of the UBV excitation beam.
FIG. 7A is a diagram of a finite element model (FEM) of a flat viscoelastic plate submerged in a fluid of the same density.
FIG. 7B a diagram of the FEM of a curved viscoelastic plate submerged in a fluid of the same density.
FIG. 8 is a diagram of a thin layer on a semi-infinite elastic medium and fluid loading according to one aspect of the present disclosure.
FIG. 9 is a plot of a Lamb wave dispersion equation fit to obtained dispersion data to estimate tissue elasticity according to one aspect of the present disclosure.
FIG. 10A is a dual axis graph of a non-limiting example Pdet and GV2 data series for DO bladders.
FIG. 10B is a dual axis graph of a non-limiting example Pdet and GV2 data series for non-DO bladders.
FIG. 11A is a graph of a non-limiting example GV2 data series for DO bladders.
FIG. 11B is another graph of a non-limiting example GV2 data series for DO bladders.
FIG. 11C is a graph of a non-limiting example GV2 data series for non-DO bladders.
FIG. 11D is another graph of a non-limiting example GV2 data series for non-DO bladders.
FIG. 12A is a graph of a non-limiting example Pdet data series for DO bladders.
FIG. 12B is another graph of a non-limiting example Pdet data series for DO bladders.
FIG. 12C is a graph of a non-limiting example Pdet data series for non-DO bladders.
FIG. 12D. is another graph of a non-limiting example Pdet data series for non-DO bladders.
FIG. 13A is a non-limiting example scatter plot of GV2 signal index against Pdet signal index.
FIG. 13B is a non-limiting example ROC curve produced by using the signal index (I) as a classifier for DO applied to GV2 data.
FIG. 13C is a non-limiting example ROC curve produced by using the signal index (I) as a classifier for DO applied to Pdet data.
FIG. 14A is a non-limiting example histogram of sampling interval statistic for bladder examples.
FIG. 14B, is another non-limiting example histogram of sampling interval statistic for bladder examples.
FIG. 14C is a non-limiting example histogram of experimental duration for each non-limiting bladder example.
FIG. 15 is a non-limiting example graph of a data series before Hampel filtering and after Hampel filtering.
FIG. 16A is a flow chart of a non-limiting example method for generating a ETP data series.
FIG. 16B shows non-limiting example graphs of a raw data series and after processing.

### DETAILED DESCRIPTION

A system and method are provided for determining viscoelasticity of the bladder, using ultrasound bladder vibrometry (UBV) to determine detrusor overactivity. UBV is a non-invasive technique that uses ultrasound radiation force that is focused to excite Lamb waves in a tissue wall and pulse-echo techniques to track the tissue deformation. In this regard, the term "bladder" may not refer to the "urinary" bladder in the human body, but may more generally refer to a tissue volume formed by tissue walls that may have some elasticity. Accordingly, the urinary bladder is one example of a bladder. Cross-spectral analysis can be used to calculate the wave velocity, which is related to the elastic properties of the bladder wall.

A time-of-flight method may be used to estimate the group velocity (GV) of the tissue from the tissue motion along the wall and the measurement may be saved and time-stamped. The method also includes generating an ensemble of transient peaks from the square of the saved and time-stamped GV measurements (GV2).

Ultrasound elastography methods have been used to measure elasticity of various soft tissues. During the past decade, several techniques based on acoustic radiation force (ARF) have been developed to study tissue elasticity. Supersonic shear imaging uses ARF to induce a shear wave in tissue and produce a map of shear elastic modulus of the tissue. More recently, Shearwave Dispersion Ultrasound Vibrometry (SDUV) has been developed for measuring tissue elasticity and viscosity.

The above techniques are generally designed assuming wave propagation in the bulk tissue (theoretically without boundaries), for applications in organs such as breast, liver, kidney, muscle and prostate. The above techniques also assume that the tissue being examined is infinitely thick. However, this assumption is not appropriate for bladders, where the expected bladder wall thickness would be at most a few millimeters. Additionally, current models in the above-mentioned methods are not appropriate for the unique properties of the bladder, as the bladder wall serves as the interface between a liquid (internally) and the rest of the body cavity (externally).

Recent studies have investigated the use of Lamb wave Dispersion Ultrasound Vibrometry (LDUV) to non-invasively quantify viscoelasticity of plate-like organs, such as the heart wall. LDUV is an SDUV-based technique that uses a mechanical actuator or ultrasound radiation force that is focused to excite Lamb waves in the medium of interest and a pulse echo transducer to track the deformation and estimate phase velocity. The Lamb wave dispersion equation is fitted to the measured Lamb wave velocity dispersion (change in phase velocity as a function of frequency) to estimate elasticity and viscosity of the medium. However, the Lamb wave dispersion equation used in LDUV assumes a flat plate model and that the plate is surrounded by water-like fluid (urine, blood, pericardial fluid, etc.) on both sides. The bladder, on the other hand, has a wall that is spatially curved and (a curved plate) and contains urine on one side, but is surrounded by connective tissue, fat and peritoneal fluid on the superficial side. Therefore, the conventionally-used LDUV technique cannot be readily applied to the bladder.

Other methods for measuring mechanical properties of the bladder, such as stiffness, include using a biosensor that directly evaluates the properties of the bladder wall. Stiffness measurements made using the biosensor can be considered to represent the instantaneous biomechanical properties of the bladder wall. The measurements can be obtained whether the bladder is empty, filling, or spontaneously contracting. Evidently, since the measurements are made only at the region of the bladder wall where the biosensor makes contact, the resulting stiffness data are more specific to that region and may be used to examine regional inhomogeneity. Although the biosensor provides a direct measure of bladder stiffness, the biosensor is mounted on a catheter that comes in contact with the bladder tissue whose stiffness is to be measured, thereby still providing an invasive procedure for the patient.

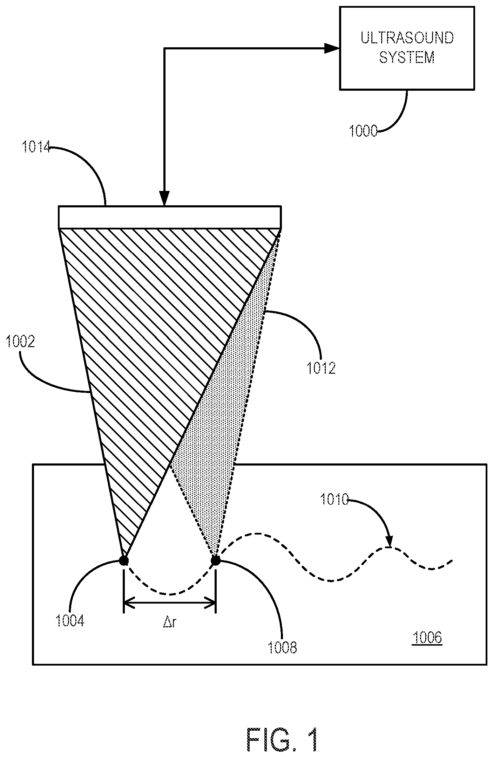

Referring particularly to FIG. 1, an ultrasound system 1000 that can implement the methods described in the present disclosure includes an ultrasonic transducer 1014 having elements that produce focused beams 1002 that may be used for delivering radiation force or imparting Lamb waves 1010 in a tissue 1006 at stimulation point 1004, and to detect tissue deformation with receiver beam 1012, such as from receiver point 1008 after a period of time Δτ. The elements may be driven by respective continuous wave synthesizers at ultrasonic frequencies that differ by a desired beat frequency. The focused beams 1002 may be aimed at a target tissue 1006 that is to be measured, and in response, the target tissue vibrates, or oscillates, with Lamb waves 1010 at a determined frequency. These elements thus serve as a force generator that oscillates the target tissues at a prescribed beat frequency.

The vibrations of the target tissue may be measured by an ultrasound system 1000. As will be described in more detail below, the ultrasound system 1000 drives an ultrasonic transducer 1014 to apply a focused or unfocused ultrasound beam 1002 to the target tissue 1006 and to receive the echo signal with receiver beam 1012 reflected by the target tissue 1006. The phase and amplitude of these echo signals may be processed as described below to measure a parameter representing a property of a tissue volume such as mechanical or tissue properties, such as group velocity (GV), group velocity squared (GV2), a power of group velocity, arrival time of the induced Lamb wave, estimated elastic properties, detrusor pressure (Pdet), detrusor activity, such as a detrusor overactivity index of the target tissue 1006, and the like.

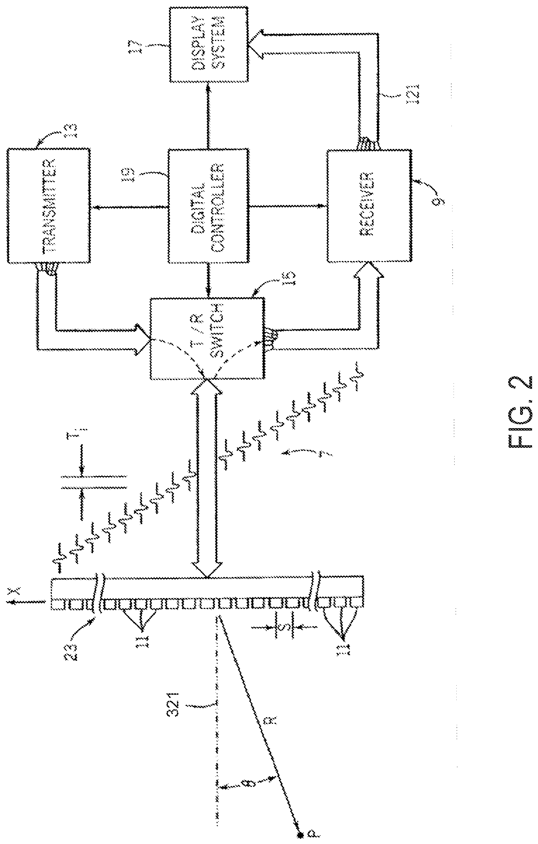

Referring particularly to FIG. 2, a transducer array 23 includes a plurality of separately driven elements 11 that each produce a burst of ultrasonic energy when energized by a pulse produced by a transmitter 13. The ultrasonic energy reflected back to the transducer array 23 from the subject under study is converted to an electrical signal by each transducer element 11 and applied separately to a receiver 9 through a set of switches 15. The transmitter 13, receiver 9 and the switches 15 are operated under the control of a digital controller 19 responsive to the commands input by the human operator. A complete scan is performed by acquiring a series of echoes in which the switches 15 are set to their transmit position, the transmitter 13 is gated on momentarily to energize each transducer element 11, the switches 15 are then set to their receive position, and the subsequent echo signals produced by each transducer element 11 are applied to the receiver 9. The separate echo signals from each transducer element 11 are combined in the receiver 9 to produce a single echo signal that is employed to produce a line in an image on a display system 17.

The transmitter 13 drives the transducer array 23 such that the ultrasonic energy produced is directed, or steered, in a beam or pulse. A B-scan can therefore be performed by moving this beam through a set of angles from point-to-point rather than physically moving the transducer array 23. To accomplish this, the transmitter 13 imparts a time delay (Tᵢ) to the respective pulses 20 that are applied to successive transducer elements 11. If the time delay is zero (Tᵢ = 0), all the transducer elements 11 are energized simultaneously and the resulting ultrasonic beam is directed along an axis 321 normal to the transducer face and originating from the center of the transducer array 23. As the time delay (Tᵢ) is increased, the ultrasonic beam is directed downward from the central axis 321 by an angle θ.

A sector scan is performed by progressively changing the time delays Tᵢ in successive excitations. The angle θ is thus changed in increments to steer the transmitted beam in a succession of directions. When the direction of the beam is above the central axis 321, the timing of the pulses 7 is reversed.

Referring still to FIG. 2, the echo signals produced by each burst of ultrasonic energy emanate from reflecting objects located at successive positions (R) along the ultrasonic beam. These are sensed separately by each transducer element 11 of the transducer array 23 and a sample of the magnitude of the echo signal at a particular point in time represents the amount of reflection occurring at a specific range (R). Due to the differences in the propagation paths between a focal point P and each transducer element 11, however, these echo signals will not occur simultaneously and their amplitudes will not be equal. The function of the receiver 9 is to amplify and demodulate these separate echo signals, impart the proper time delay to each and sum them together to provide a single echo signal that accurately indicates the total ultrasonic energy reflected from each focal point P located at range R along the ultrasonic beam oriented at the angle θ.

To simultaneously sum the electrical signals produced by the echoes from each transducer element 11, time delays are introduced into each separate transducer element channel of the receiver 9. In the case of the linear array 23, the delay introduced in each channel may be divided into two components, one component is referred to as a beam steering time delay, and the other component is referred to as a beam focusing time delay. The beam steering and beam focusing time delays for reception are precisely the same delays (Tᵢ) as the transmission delays described above. However, the focusing time delay component introduced into each receiver channel is continuously changing during reception of the echo to provide dynamic focusing of the received beam at the range R from which the echo signal emanates.

Under the direction of the digital controller 19, the receiver 9 provides delays during the scan such that the steering of the receiver 9 tracks with the direction of the beam steered by the transmitter 13 and it samples the echo signals at a succession of ranges and provides the proper delays to dynamically focus at points P along the beam. Thus, each emission of an ultrasonic pulse results in the acquisition of a series of data points that represent the amount of reflected sound from a corresponding series of points P located along the ultrasonic beam.

By selecting proper time delays, echoes from multiple focused locations can be received to measure vibration information from several points of the tissue. The limitation of the lateral resolution of the transducer for two closely located points can be improved by assigning different transmitting codes for different locations.

The display system 17 receives the series of data points produced by the receiver 9 and converts the data to a form producing the desired image. For example, if an A-scan is desired, the magnitude of the series of data points is merely graphed as a function of time. If a B-scan is desired, each data point in the series is used to control the brightness of a pixel in the image, and a scan comprised of a series of measurements at successive steering angles (θ) is performed to provide the data that can be used for display of an image.

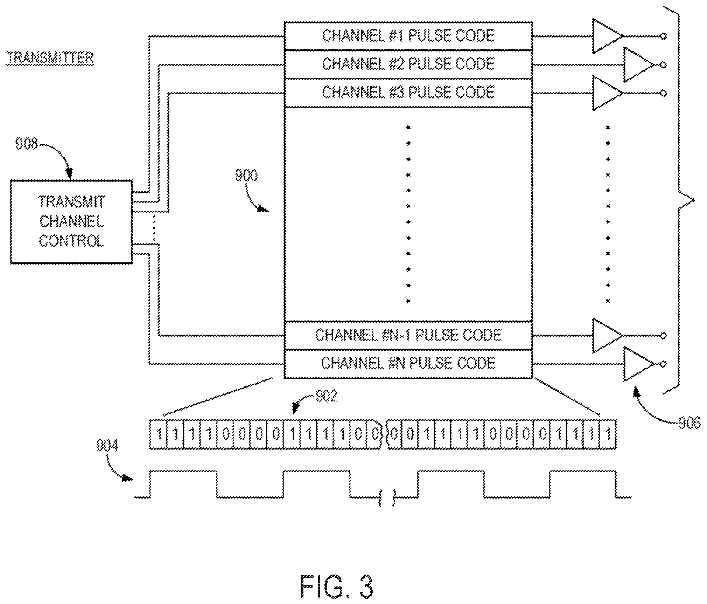

Referring particularly to FIG. 3, the transmitter 13 includes a set of channel pulse code memories that are indicated collectively at 900. Each pulse code memory 900 stores a bit pattern 902 that determines the frequency of the ultrasonic pulse 904 that is to be produced. This bit pattern is read out of each pulse code memory 900 by a master clock and applied to a driver 906 that amplifies the signal to a power level suitable for driving the transducer 23. In the example shown in FIG. 3, the bit pattern is a sequence of four "1" bits alternated with four "0" bits to produce a 5 megahertz ("MHz") ultrasonic pulse 904. The transducer elements 23 to which these ultrasonic pulses 904 are applied respond by producing ultrasonic energy.

As indicated above, to steer the transmitted beam of the ultrasonic energy in the desired manner, the pulses 904 for each of the N channels are produced and delayed by the proper amount. These delays are provided by a transmit control 908 that receives control signals from the digital controller 19. When the control signal is received, the transmit control 908 gates a clock signal through to the first transmit channel. At each successive delay time interval thereafter, the clock signal is gated through to the next channel pulse code memory 900 until all the channels to be energized are producing their ultrasonic pulses 904. Each transmit channel may be reset after its entire bit pattern 902 has been transmitted and the transmitter 13 then waits for the next control signal from the digital controller 19.

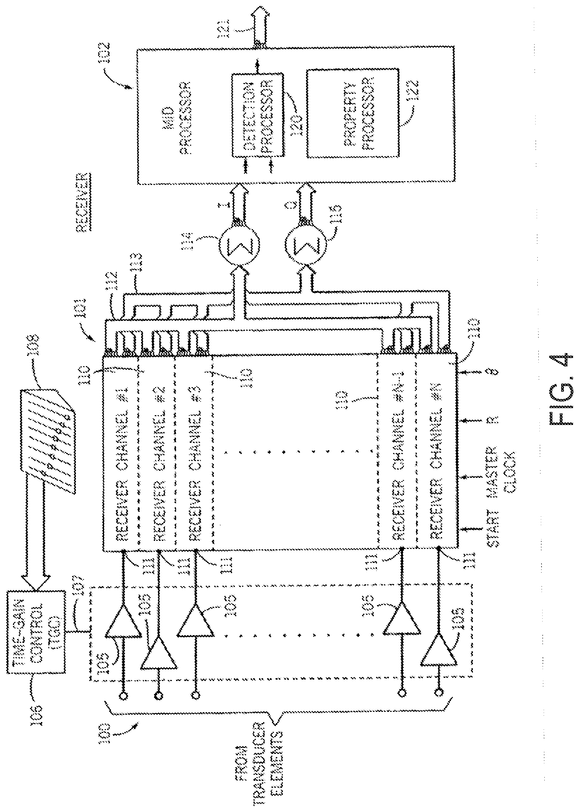

Referring particularly to FIG. 4, the receiver 9 is comprised of three sections: a time-gain control section 100, a beam forming section 101, and a mid processor 102. The time-gain control section 100 includes an amplifier 105 for each of the N=128 receiver channels and a time-gain control circuit 106. The input of each amplifier 105 is connected to a respective one of the transducer elements 11 to receive and amplify the echo signal that it receives. The amount of amplification provided by the amplifiers 105 is controlled through a control line 107 that is driven by the time-gain control circuit 106. As is well known in the art, as the range of the echo signal increases, its amplitude is diminished. As a result, unless the echo signal emanating from more distant reflectors is amplified more than the echo signal from nearby reflectors, the brightness of the image diminishes rapidly as a function of range (R). This amplification is controlled by the operator who manually sets TGC linear potentiometers 108 to values that provide a relatively uniform brightness over the entire range of the sector scan. The time interval over which the echo signal is acquired determines the range from which it emanates, and this time interval is divided into by the TGC control circuit 106. The settings of the potentiometers are employed to set the gain of the amplifiers 105 during each of the respective time intervals so that the echo signal is amplified in ever increasing amounts over the acquisition time interval.

The beam forming section 101 of the receiver 9 includes N=128 separate receiver channels 110. Each receiver channel 110 receives the analog echo signal from one of the TGC amplifiers 105 at an input 111, and it produces a stream of digitized output values on an I bus 112 and a Q bus 113. Each of these I and Q values represents a sample of the echo signal envelope at a specific range (R). These samples have been delayed in the manner described above such that when they are summed at summing points 114 and 115 with the I and Q samples from each of the other receiver channels 110, they indicate the magnitude and phase of the echo signal reflected from a point P located at range R on the steered beam (θ).

Referring still to FIG. 4, the mid processor section 102 receives the beam samples from the summing points 114 and 115. The I and Q values of each beam sample are a 16-bit digital number that represents the in-phase and quadrature components of the magnitude of the reflected sound from a point (R,θ). The mid processor 102 can perform a variety of calculations on these beam samples, where choice is determined by the type of image to be reconstructed.

For example, a conventional ultrasound image may be produced by a detection processor 120 that calculates the magnitude of the echo signal from its I and Q components: $M = \sqrt{{I}^{2} + {Q}^{2}} .$

The resulting magnitude values output at 121 to the display system 17 result in an image in which the magnitude of the reflected echo at each image pixel is indicated.

The present methods may be implemented by a parameter representing a property of a tissue volume processor, or property processor 122 that forms part of the mid-processor 102. As will be explained in detail below, this processor 102 receives the I and Q beam samples acquired during a sequence of measurements of the subject tissue and calculates a mechanical property (i.e., thickness, activity, and the like) of the tissue.

In accordance with the present disclosure, methods are provided for detecting DO through analysis of transient peaks in UBV measurements. Peaks may be characterized in these measurements through a DO index (*I*) that characterizes the differing signal energies between peaks and lower envelope (LE). A statistically significant difference in I between DO and non-DO bladders was observed in the non-limiting examples provided.

In some configurations, GV2 data series may be gathered from subjects, such as subjects with neurogenic bladder conditions. Acquired data may be processed to identify DO associated transient peaks by decomposing the data series into ETS and LE series. In order to characterize the decomposed series by a single number, a DO index, *I*, may be determined.

In order to characterize fluctuations in GV2 measurement series associated with DO, such fluctuations may be separated from any larger overall trend associated with bladder filling. Fluctuations that are not likely to be a result of bladder wall activity and that can dominate the characterization, such as large outlier measurements may be removed. For the latter consideration, a statistical filtering approach (e.g. Hampel filtering) may be used. To separate DO associated fluctuations from the main trend of the series, the series may be decomposed into LE and ETP series respectively.

Prior to peak identification, values of GV2 in which a bladder leak is reported may be removed from the given data series. Measurements at the final volume of the experiment may also be excluded from the peak identification process in order to avoid the inclusion of normal detrusor contraction associated with the onset of voiding. Such outliers could be due to, for instance, patient motion during measurement or unusually weak UBV signals. In order to remove these outliers, a statistical outlier filter, such as a Hampel filter, may be applied to the GV2 data series. This filter works by replacing points determined to be outliers with the median of the sample window. The filter may identify outliers such as a given sample from the data series, xᵢ, which is the center point of sliding window with a median of mᵢ, if $\left|{x}_{i}-{m}_{i}\right| > {n}_{σ} {σ}_{i}$

Here, n_{σ} is a threshold number of standard deviations and σᵢ is the estimated standard deviation of the sampling window. The standard deviation of the sampling window may be estimated from the median absolute deviation as: ${σ}_{i} = kmedian \left(\left|{x}_{i - w}-{m}_{i}\right|,…,\left|{x}_{i + w}-{m}_{i}\right|\right)$

Where w is the number of pixels on either side of the centerpoint of the window and the parameter, k, scales the median absolute deviation to the standard deviation.

After outlier filtering, the resulting data series may be decomposed into ETP and LE series. A smoothing filter may be applied to the raw data series (x) to capture any trend in the data series associated with bladder filling. Smoothing may be applied to the series through a filter such as a 2^{nd}-order Savitzky-Golay filter. The Savitzky-Golay filter applies a (2^{nd} order) polynomial regression to data within a sliding window. Because the time intervals may be irregularly spaced, a smoothing factor may be used in place of a discrete window size. The smoothing factor may be selected to take on a value between 0 and 1, which corresponds to the percent of signal energy being attenuated by smoothing (e.g. a smoothing factor of 0.5 would attenuate 50% of the signal energy). The smoothing factor may be set to a strong value, such as 0.95, in order to filter all fluctuations in the data series except for the main trend of the data.

After smoothing, x may be differenced with the filtered data series (xₛ) and time-points associated with negative values of the differenced series may be identified (i.e. time points were (x-xₛ)<0). The start and end time-points may also be included in the identified time points so that increases at the time boundary are not treated as transient peaks.

Values from x at the identified time-points may then be collected. Linear interpolation may be used to assign values at the time-points that were not identified. The resulting LE series (x_{LE}) may have the same number of elements as x. The ETP series (x_{ETP}) may then be determined by differencing the x_{LE} from x. The ETP and LE series can then be used for further analysis.

### Diagnostic Metric

In order to make comparisons between DO and non-DO bladders relative to the peaks, a single metric that characterizes the identified transient peaks may be used. Additionally, such metric should be, in principle, comparable from experiment to experiment given the variable experimental durations and ranges of UBV measurements previously noted. This metric, called detrusor overactivity (DO) index, is denoted by *I*: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}}$

Here x_{ETP}(t) denotes the continuous signal of the ETP data series and x_{LE}(t) the continuous signal of the LE data series, each as a function of time, for a given experiment. Essentially, *I* is the square root of the ratio of signal energies (denoted as E_{ETP} and E_{LE} respectively) from the transient peaks and the lower envelope. As such, it is expected that data series from bladders with DO will have a larger signal energy in x_{ETP}(t) relative to x_{LE}(t), and thus a larger *I* than bladders without DO. Because *I* is unitless, it is reasonable to make comparisons between experiments as longer experimental durations and wider ranges in measured GV2 or Pdet are accounted for by normalizing RMS(x_{ETP}) by RMS(x_{LE}).

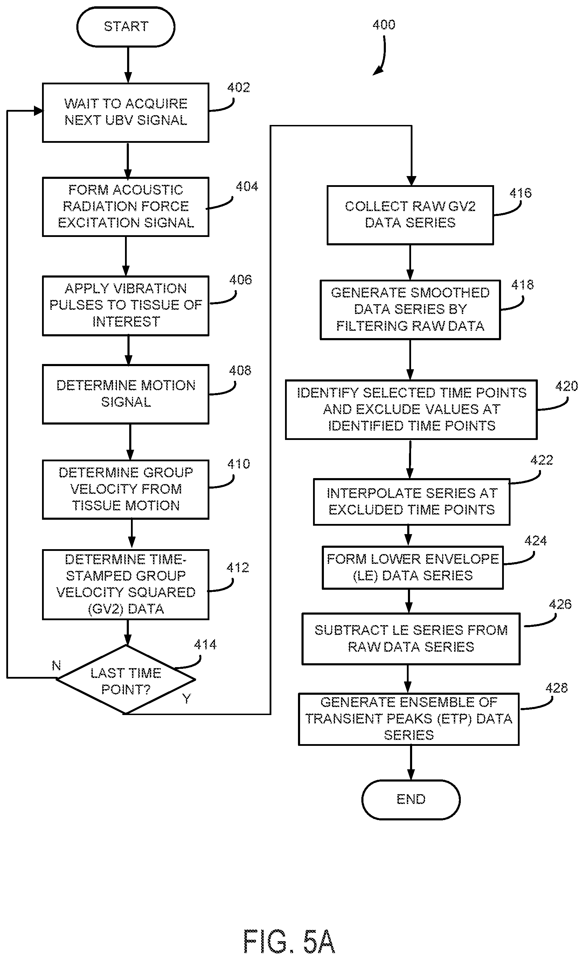

Referring to FIG. 5A, a flowchart of a non-limiting example method 400 is shown for determining detrusor overactivity. At step 402, the system may wait to acquire the next measurement, such as for a specified period of time. An acoustic radiation force excitation signal may be formed at step 404 and vibration pulses may be applied to a tissue of interest at step 406. A motion signal may be determined at step 408, where a pulse-echo ultrasound (detection beam) is used to measure the motion at several points along a line of propagation, the line of propagation being, for example, a bladder wall, in order to track tissue motion through successive B-modes.

The Group Velocity is determined at step 410 by measuring the propagation distance of an impulse as a function of time and distance. The slope of that line is the group velocity. A non-limiting example method to measure the group velocity includes measuring the time that takes for the peak of the pulse to travel a known distance. This can be done by monitoring the pulse propagation in tissue. The monitoring can be done by correlating the consecutive A-line (radio frequency echo-signals) in a B-mode ultrasound of the region of interest. The time it takes the pulse to travel from its origin (where the push beam was exerted) to a point at a known distance from where the origin is measured. In another non-limiting example, one can measure the time that takes the pulse to pass from one point in the object to another. To obtain more accurate measurements, the process can be repeated at multiple points and the group velocity can be estimated as the slope of the regression line in a distance vs. time plot. At step 412, the Group Velocity is squared to form the GV2 data series, time stamped, and saved for further processing.

A time stamped data series of raw GV2 data series may be collected or aggregated from the applied excitation at step 416, once the last time point has been reached at step 414. A smoothed data series may be generated at step 418 by filtering the raw data. Select time points may be identified at step 420 and excluded from the series. The series may be interpolated at the excluded time points at step 422. A lower envelope (LE) data series may be formed at step 424. The LE series may be subtracted from the raw data series at step 426. Energies from the ETP data series may then be generated at step 428.

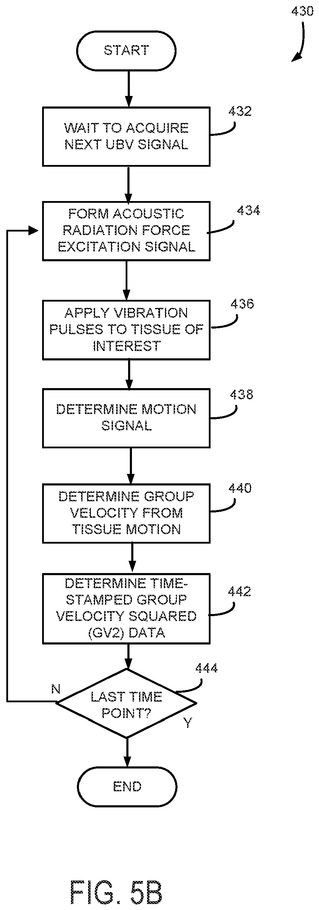

Referring to FIG. 5B, a flowchart of a non-limiting example method 430 is shown for determining detrusor overactivity. At step 432, the system may wait to acquire the next measurement, such as for a specified period of time. An acoustic radiation force excitation signal may be formed at step 434 and vibration pulses may be applied to a tissue of interest at step 436. A motion signal may be determined at step 438, where a pulse-echo ultrasound (detection beam) is used to measure the motion at several points along a line of propagation, the line of propagation being, for example, a bladder wall, in order to track tissue motion through successive B-modes. The Group Velocity is determined at step 440. At step 442, the Group Velocity is squared to form the GV2 data series, time stamped, and saved for further processing once the last time point has been reached at step 444. If the last time point has not been reached, the method may restart at step 434 to reform an acoustic radiation force excitation signal.

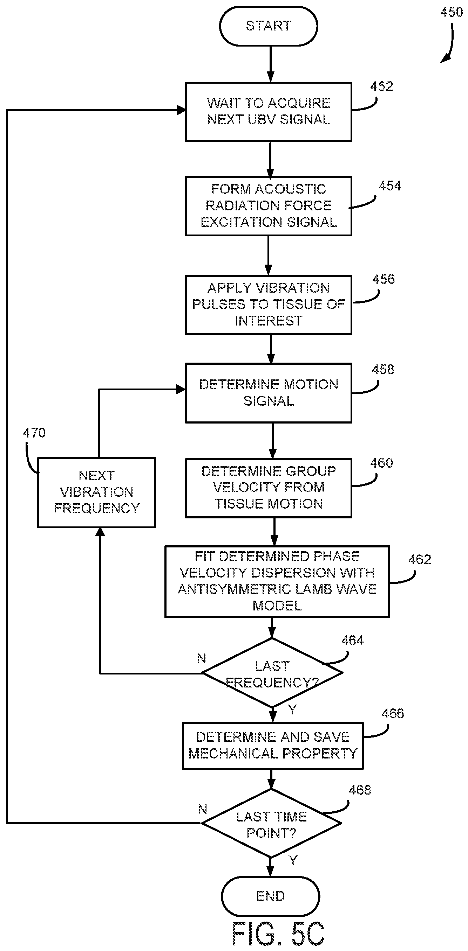

Referring to FIG. 5C, a flowchart of a non-limiting example method 450 is shown for determining detrusor overactivity. At step 452, the system may wait to acquire the next UBV measurement, such as for a specified period of time. An acoustic radiation force excitation signal may be formed at step 454 and vibration pulses may be applied to a tissue of interest at step 456. A motion signal may be determined at step 458, where a pulse-echo ultrasound (detection beam) is used to measure the motion at several points along a line of propagation, the line of propagation being, for example, a bladder wall, in order to track tissue motion through successive B-modes. The Group Velocity is determined at step 460. At step 462, phase velocity dispersion data may be determined and fit with an antisymmetric Lamb wave model, which may be saved for further processing once the last frequency has been reached at step 464. If the last frequency has not been reached, the method may include determining a subsequent vibration frequency at step 470 and restarting at step 458 to determine a new motion signal. If the last frequency is reached at step 464, a mechanical property may be determined and saved at step 466. If a last time point is reached at step 468, the method may conclude, if not, then the method may restart at step 452 to wait and acquire a next UBV signal.

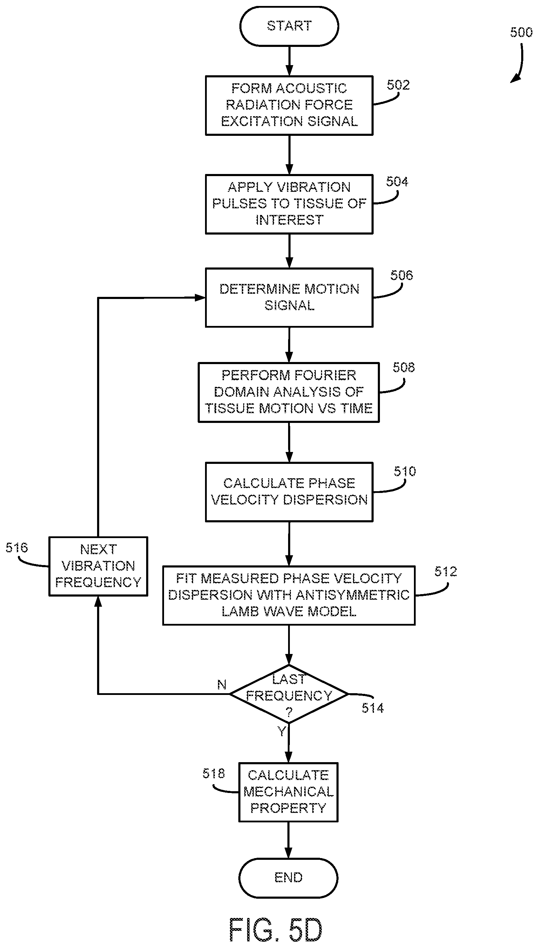

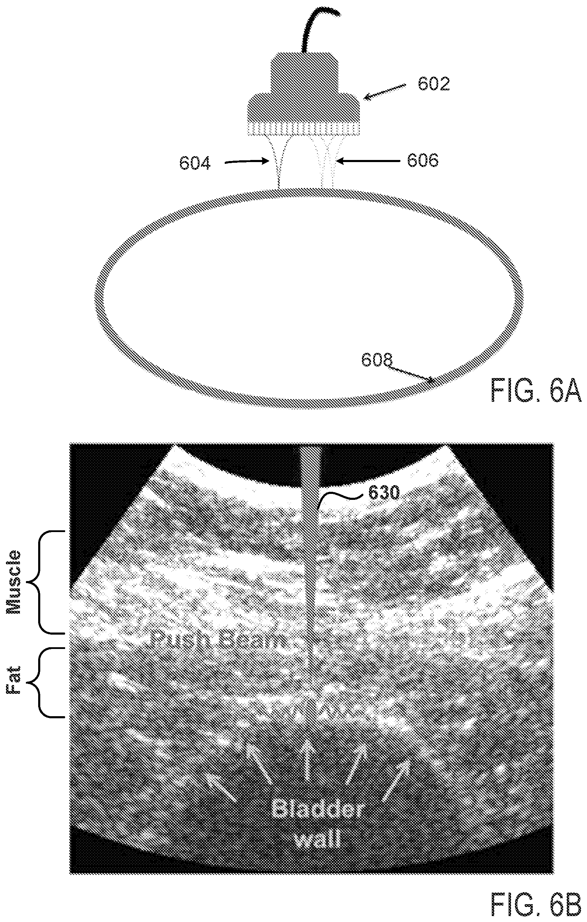

Referring now to FIG. 5D, a flow chart is provided setting forth example steps 500 of a method to measure one or more mechanical properties of tissue 21. The process begins at process block 502 with the formation of acoustic radiation force excitation signals that are applied, at process block 504, to a subject to, thereby, apply vibration pulses to tissue of interest. Referring to FIG. 6, a diagram of the fundamental principal of ultrasound bladder vibrometry (UBV) is shown. Again, the term "bladder" may not refer to the "urinary" bladder in the human body, but may, more generally, refer to a tissue volume formed by tissue walls that may have some elasticity. UBV uses focused ultrasound to produce a radiation force 604 (push beam) to excite impulsive Lamb waves (200-600 µs in length) in the medium of interest. The radiation force excitation 604 can be, for example, of 600 µs toneburst.

The focused ultrasound radiation force applied on a membrane-like medium, such as the bladder wall, excites cylindrical waves. The urine filled bladder can be modeled as an incompressible, homogenous, isotropic solid submerged in an incompressible non-viscous fluid, for example. The anti-symmetric Lamb wave dispersion equation (2) for such geometry is as follows: $4 {k}_{L}^{2} ηβ cosh \left(ηh\right) sinh \left(βh\right) - {\left(2{k}_{L}^{2}-{k}_{s}^{2}\right)}^{2} sinh \left(ηh\right) cosh \left(βh\right) = \frac{{ρ}_{1} {ηk}_{s}^{4}}{{ρ}_{2} {η}_{f}} cosh \left(ηh\right) cosh \left(βh\right)$

Where $β = \sqrt{{k}_{L}^{2} - {k}_{s}^{2}} , η = \sqrt{{k}_{L}^{2} - {k}_{p}^{2}} , {η}_{f} = \sqrt{{k}_{L}^{2} - {k}_{f}^{2}}$*, k_{f}* is the wave number of the compressional wave in the fluid, *kₚ* is the wave number of the compressional wave in tissue, *k_{L}* = *ω*/*c_{L}* is the Lamb wave number, *ω* is the angular frequency, *c_{L}* is the frequency dependent Lamb wave velocity, ${k}_{S} = ω \sqrt{{ρ}_{m} / μ}$ is the shear wave number, *ρₘ* is the density and *h* is the half-thickness of the bladder wall (*H =* 2*h*). For the case of tissue, urine and blood, the compressional wave numbers (*kₚ* and *k_{f}*) are similar and much smaller than the shear and Lamb wave numbers (*kₛ*) and (*k_{L}*), so ${η}_{f} \approx η = \sqrt{{k}_{L}^{2} - {k}_{p}^{2}} \approx {k}_{L}$. Due to similar densities of tissue, urine and blood, the dispersion equation (2) is simplified as: $\begin{matrix}4 {k}_{L}^{3} β cosh \left({k}_{L}h\right) sinh \left(βh\right) - {\left({k}_{s}^{2}-2{k}_{L}^{2}\right)}^{2} sinh \left({k}_{L}h\right) cosh \left(βh\right) \\ = {k}_{s}^{4} cosh \left({k}_{L}h\right) cosh \left(βh\right)\end{matrix}$

Alternatively, an external mechanical vibrator (not shown) can be used to excite shear waves in the bladder. With the external mechanical vibrator, the ultrasound radiation force is not applied, and in these instances ultrasound is used to detect the waves on the bladder wall and not to generate the shear waves within the bladder. The mechanical vibrator may include a speaker-like mechanism with a plastic shaft that transfers the mechanical energy to the skin. The mechanical energy then vibrates the abdomen and the bladder.

Referring again to FIGS. 5D and 6, at process block 506, a detection beam 606 (e.g., pulse-echo ultrasound) is used to measure the motion at several points along a line of propagation 608, the line of propagation being, for example, a bladder wall, in order to track tissue motion through successive B-modes. Though the following description is made with respect to the bladder wall, other elastic tissue volumes may likewise be considered. The B-mode scanning can be performed by, for example, a Verasonics ultrasound imaging platform equipped with a C4-2 linear array transducer 602, as shown in FIG. 6A, to excite 400-600 µs impulse in the bladder wall and track the mechanical motion. Verasonics is a registered trademark of Verasonics, Inc. Corporation of Redmond, WA. The detection beam 606 can be transmitted with a pulse repetition frequency (PRF) of about 2.0 kHz for example (2.5 kHz in another example) with a center of frequency of about 3.0 MHz (3.3 MHz in a specific case). FIG. 6B illustrates *in vivo* human B-mode of the bladder with a sketch of the UBV excitation beam 630.

Cross-spectral analysis of the received echoes can then be used to calculate the bladder wall motion as a function of time. Because an impulse contains mostly the frequency components up to the inverse of the time length of the impulse, many phase velocities from a single impulse push can be extracted. As shown at process block 508, a two-dimensional fast Fourier transform (2D-FFT) of the bladder wall motion may be used to calculate the change of Lamb wave velocity as a function of frequency, or the Lamb wave dispersion. In other words, Fourier-space analysis of the motion may be used as a function of time that yields the k-space whose coordinates are frequency, *f*, and wave number, *k.* Since the wave velocity c = *k*/*f,* the phase velocity at each frequency can be calculated at process block 510 by searching for peaks at the given frequency and dividing by the wave number coordinate by the frequency coordinate for the given peak. The Lamb wave dispersion equation may be fit to the dispersion data at process block 512 to estimate bladder viscoelasticity, as shown at process block 518. As indicated by process block 514, the digital controller determines whether the last frequency has been measured when a harmonic excitation approach is used. If not, at process block 516, another frequency is selected and process blocks 506 through 512 are repeated at each desired prescribed frequency.

As discussed above, the Lamb wave model assumes that the bladder is surrounded by a fluid on both sides, which, in some situations, may not be sufficient to quantify mechanical properties of the bladder. In accordance with another aspect of the present disclosure, a model can be derived to account for the fact that the bladder wall is surrounded by urine on one side and a soft connective tissue and muscle on the other.

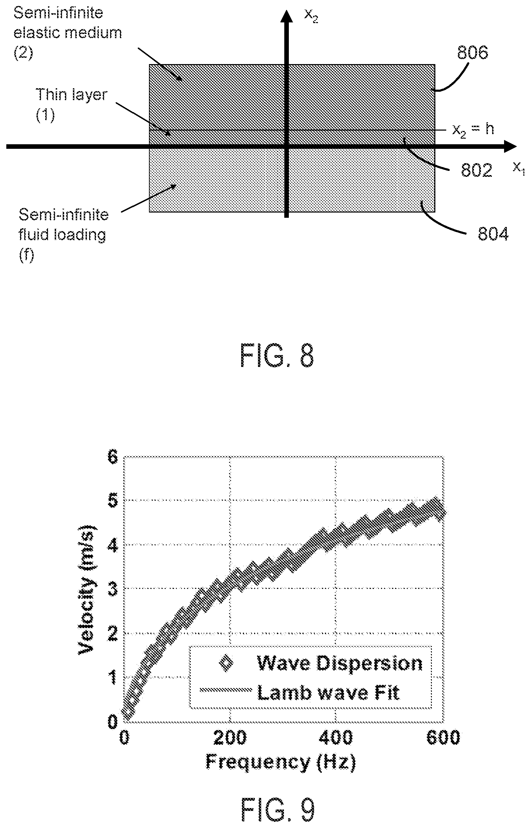

Referring to FIG. 8, an analytical model for Lamb wave propagation can be created that includes a plate or thin layer 802 surrounded by a semi-infinite fluid 804 on one side and a semi-infinite solid 806 on the other. This model may be advantageous because it mimics the natural surroundings of the bladder wall. The model includes a thin layer 802 of thickness, h, in contact with a rigid half space on one side and fluid coupled to the upper surface as shown in FIG. 8. The potential functions for the wave motion can be described as follows: $\begin{matrix}{ϕ}_{1} = \left({a}_{1}{e}^{- {iη}_{1} {x}_{2}}+{b}_{1}{e}^{{iη}_{1} {x}_{2}}\right) {e}^{{ikx}_{1}} \\ {ψ}_{1} = \left({c}_{1}{e}^{- {iβ}_{1} {x}_{2}}+{d}_{1}{e}^{{iβ}_{1} {x}_{2}}\right) {e}^{{ikx}_{1}} \\ {ϕ}_{2} = {b}_{2} {e}^{- {η}_{2} {x}_{2}} {e}^{{ikx}_{1}} \\ {ψ}_{2} = {d}_{2} {e}^{- {β}_{2} {x}_{2}} {e}^{{ikx}_{1}} \\ {ϕ}_{f} = {me}^{{η}_{f} {x}_{2}} {e}^{{ikx}_{1}}\end{matrix}$
where ${η}_{1} = \sqrt{{k}_{p 1}^{2} - {k}^{2}} , {β}_{1} = \sqrt{{k}_{s 1}^{2} - {k}^{2}} , {η}_{2} = \sqrt{{k}^{2} - {k}_{p 2}^{2}} , {β}_{2} = \sqrt{{k}^{2} - {k}_{s 1}^{2}} , {η}_{f} = \sqrt{{k}^{2} - {k}_{f}^{2}}$, and *k is* the wave number, a subscript *p* denotes the primary or compression wave, a s subscript denotes the shear wave, *f* denotes the fluid. The following boundary conditions can be applied at x₂ = *h:* $\begin{matrix}{σ}_{22}^{1} = {σ}_{22}^{2} \\ {σ}_{12}^{1} = {σ}_{12}^{2} \\ {u}_{1}^{1} = {u}_{1}^{2} \\ {u}_{2}^{1} = {u}_{2}^{2}\end{matrix} ,$
and the following boundary conditions at the interface between the layer and rigid half-space *x*₂ = 0: $\begin{matrix}{σ}_{22}^{1} = {σ}_{22}^{f} \\ {σ}_{12}^{1} = 0 \\ {u}_{2}^{1} = {u}_{2}^{f}\end{matrix} .$

Applying the boundary conditions above with the potential functions in (4), the plane stress in the rigid substrate at *x*₂ = *h,* where *E*₁ = *e*^{*iη*₁}*^{h}, B*₁ *= e*^{*iβ*₁}*^{h}, E*₂ *= e*^{*-η*₂*h*}, *B*₂ *= e*^{*-β*₂*h*}, can be described as follows: $\begin{matrix}{σ}_{22}^{1} = {σ}_{22}^{2} \\ {σ}_{22}^{1} = - {μ}_{1} \left\{{k}_{s 1}^{2}{ϕ}_{1}+2\left({ψ}_{1 , 12}+{ϕ}_{1 , 11}\right)\right\} \\ {σ}_{22}^{2} = - {μ}_{2} \left\{{k}_{s 2}^{2}{ϕ}_{2}+2\left({ψ}_{2 , 12}+{ϕ}_{2 , 11}\right)\right\} \\ {σ}_{22}^{1} = - {μ}_{1} \left\{\left[{k}_{s 1}^{2}-{k}^{2}\right]\left({a}_{1}{E}_{1}^{- 1}+{b}_{1}{E}_{1}\right)+2{kβ}_{1}\left({c}_{1}{B}_{1}^{- 1}-{d}_{1}{B}_{1}\right)\right\} {e}^{{ikx}_{1}} \\ {σ}_{22}^{2} = - {μ}_{2} \left\{\left[{k}_{s 2}^{2}-{k}^{2}\right]{b}_{2}{E}_{2}-\left.2{kβ}_{2}{d}_{2}{B}_{2}\right)\right\} {e}^{{ikx}_{1}}\end{matrix}$

The shear stress at *x*₂ = 0 is: $\begin{matrix}{σ}_{12}^{1} = 0 \\ {σ}_{12}^{1} = {μ}_{1} \left\{2{ϕ}_{1 , 12}+{ψ}_{1 , 22}-{ψ}_{1 , 11}\right\} \\ {σ}_{12}^{1} = {μ}_{1} \left\{2{kη}_{1}\left({a}_{1}-{b}_{1}\right)+\left({k}^{2}+{β}_{1}^{2}\right)\left({c}_{1}+{d}_{1}\right)\right\} {e}^{{ikx}_{1}} = 0\end{matrix}$

The shear stress at *x*₂ = h is: $\begin{matrix}{σ}_{12}^{1} = {σ}_{12}^{2} \\ {σ}_{12}^{1} = {μ}_{1} \left\{2{ϕ}_{1 , 12}+{ψ}_{1 , 22}-{ψ}_{1 , 11}\right\} \\ {σ}_{12}^{2} = {μ}_{2} \left\{2{ϕ}_{2 , 12}+{ψ}_{2 , 22}-{ψ}_{2 , 11}\right\} \\ {σ}_{12}^{1} = {μ}_{1} \left\{2{kη}_{1}\left({a}_{1}{E}_{1}^{- 1}-{b}_{1}{E}_{1}\right)+\left({k}^{2}-{β}_{1}^{2}\right)\left({c}_{1}{B}_{1}^{- 1}+{d}_{1}{B}_{1}\right)\right\} {e}^{{ikx}_{1}} \\ {σ}_{12}^{1} = {μ}_{2} \left\{-2{ikη}_{2}{b}_{2}{E}_{2}+\left({k}^{2}+{β}_{2}^{2}\right){d}_{2}{B}_{2}\right\} {e}^{{ikx}_{1}}\end{matrix}$

The displacements along the *x*₁ direction due to the boundary condition at *x*₂ *= h:* $\begin{matrix}{u}_{1}^{1} = {u}_{1}^{2} \\ {u}_{1}^{1} = \left\{{ϕ}_{1 , 1}+{ψ}_{1 , 2}\right\} \\ {u}_{1}^{2} = \left\{{ϕ}_{2 , 1}+{ψ}_{2 , 2}\right\} \\ {u}_{1}^{1} = \left\{ik\left({a}_{1}{E}_{1}^{- 1}+{b}_{1}{E}_{1}\right)+{iβ}_{1}\left(-{c}_{1}{B}_{1}^{- 1}+{d}_{1}{B}_{1}\right)\right\} {e}^{{ikx}_{1}} \\ {u}_{1}^{2} = \left\{{ikb}_{2}{E}_{2}-\left.{β}_{2}{d}_{2}{B}_{2}\right)\right\} {e}^{{ikx}_{1}}\end{matrix}$

The plane stress in the substrate due to fluid loading at *x*₂ = 0 is: $\begin{matrix}{σ}_{22}^{1} = {σ}_{22}^{f} \\ {σ}_{22}^{1} = - {μ}_{1} \left\{{k}_{s 1}^{2}{ϕ}_{1}+2\left({ψ}_{1 , 12}+{ϕ}_{1 , 11}\right)\right\} \\ {σ}_{22}^{f} = - {μ}_{f} \left\{{ϕ}_{2 , 11}\right\} \\ {σ}_{22}^{1} = - {μ}_{1} \left\{\left[{k}_{s 1}^{2}-{k}^{2}\right]\left({a}_{1}+{b}_{1}\right)+2{kβ}_{1}\left({c}_{1}-{d}_{1}\right)\right\} {e}^{{ikx}_{1}} \\ {σ}_{22}^{f} = {ρ}_{f} {ω}^{2} {me}^{{n}_{f} {x}_{2}} {e}^{{ikx}_{1}} = \frac{{μ}_{1} {ρ}_{f} {k}_{s 1}^{2}}{{ρ}_{1}} {me}^{{ikx}_{1}}\end{matrix}$

The displacement in the x₂ direction due to fluid loading at *x*₂ = 0 is: $\begin{matrix}{u}_{2}^{1} = {u}_{2}^{f} \\ {u}_{2}^{1} = \left\{{ϕ}_{1 , 2}-{ψ}_{1 , 1}\right\} \\ {u}_{2}^{f} = {ϕ}_{2 , 2} \\ {u}_{2}^{1} = \left\{{iη}_{1}\left(-{a}_{1}+{b}_{1}\right)+ik\left({c}_{1}+{d}_{1}\right)\right\} {e}^{{ikx}_{1}} \\ {u}_{2}^{f} = {n}_{f} {me}^{{n}_{f} {x}_{2}} {e}^{{ikx}_{1}} = {n}_{f} {me}^{{ikx}_{1}}\end{matrix}$

The displacement in the *x*₂ direction due to fluid loading at *x*₂ = h is: $\begin{matrix}{u}_{2}^{1} = {u}_{2}^{2} \\ {u}_{2}^{1} = \left\{{ϕ}_{1 , 2}-{ψ}_{1 , 1}\right\} \\ {u}_{2}^{2} = \left\{{ϕ}_{2 , 2}+{ψ}_{2 , 1}\right\} \\ {u}_{2}^{1} = \left\{{iη}_{1}\left(-{a}_{1}{E}_{1}^{- 1}+{b}_{1}{E}_{1}\right)+ik\left({c}_{1}{B}_{1}^{- 1}+{d}_{1}{B}_{1}\right)\right\} {e}^{{ikx}_{1}} \\ {u}_{2}^{2} = \left\{-{η}_{2}{b}_{2}{E}_{2}\left.-{ikd}_{2}{B}_{2}\right)\right\} {e}^{{ikx}_{1}}\end{matrix}$

Assembling these equations into a system of equations results in matrix *A* below. To obtain non-trivial results, the determinant of *A* is set to zero (det(*A*) = 0). Using these results the dispersion curves for specified frequency ranges can be plotted. $A = \left[\begin{matrix}\left({k}_{s 1}^{2}-{k}^{2}\right) & \left({k}_{s 1}^{2}-{k}^{2}\right) & 2 {kβ}_{1} & - 2 {kβ}_{1} & 0 & 0 & {k}_{s 1}^{2} \\ - {iη}_{1} & {iη}_{1} & - ik & - ik & 0 & 0 & {n}_{f} \\ 2 {kη}_{1} & - 2 {kη}_{1} & \left(2{k}^{2}-{k}_{s 1}^{2}\right) & \left(2{k}^{2}-{k}_{s 1}^{2}\right) & 0 & 0 & 0 \\ \left(2{k}^{2}-{k}_{s 1}^{2}\right) {μ}_{1} {E}_{1}^{- 1} & \left(2{k}^{2}-{k}_{s 1}^{2}\right) {μ}_{1} {E}_{1} & - 2 {kβ}_{1} {B}_{1}^{- 1} & - 2 {kβ}_{1} {B}_{1} & - \left(2{k}^{2}-{k}_{s 2}^{2}\right) {μ}_{2} {B}_{2} & - 2 {ikβ}_{2} {μ}_{2} {B}_{2} & 0 \\ 2 {kη}_{1} {μ}_{1} {E}_{1}^{- 1} & - 2 {kη}_{1} {μ}_{1} {E}_{1} & \left(2{k}^{2}-{k}_{s 1}^{2}\right) {μ}_{1} {B}_{1}^{- 1} & \left(2{k}^{2}-{k}_{s 1}^{2}\right) {μ}_{1} {B}_{1} & 2 {kη}_{2} {μ}_{2} {E}_{2} & - \left(2{k}^{2}-{k}_{s 2}^{2}\right) {μ}_{2} {B}_{2} & 0 \\ {ikE}_{1}^{- 1} & {ikE}_{1} & - {iβ}_{1} {B}_{1}^{- 1} & {iβ}_{1} {B}_{1} & - {ikE}_{2} & {β}_{2} {B}_{2} & 0 \\ - {iη}_{1} {E}_{1}^{- 1} & {iη}_{1} {E}_{1} & - {ikB}_{1}^{- 1} & - {ikB}_{1} & {η}_{2} {E}_{2} & {ikB}_{2} & 0\end{matrix}\right]$

The dispersion equation (14) can be used to quantify mechanical properties of the bladder wall, similar to the aforementioned Lamb wave dispersion described with respect to equation (2). The determinant of matrix A is set to zero and solved numerically to obtain the desired dispersion curves. Using the dispersion matrix A in UBV accounts for the media surrounding the bladder wall and provides a more complete description of the stresses and strains that affect the wave propagation in the bladder wall due to radiation force.

In addition, the bladder tissue can experience both geometric attenuation (this attenuation occurs due to boundaries in the geometry) and attenuation due to material viscosity (this attenuation would occur even if the medium was infinite). The geometric attenuation may be accounted for by using the anti-symmetric Lamb wave model, which shows significant dispersion in plates even in the absence of viscosity. The attenuation due to viscosity is accounted for by using the Voigt model to describe tissue elasticity and viscosity as two separate factors. In the case of the bladder wall, the attenuation can be significant, and the waves can die out within a few centimeters from the excitation point, thereby ignoring the possible reflections and wrapping of the waves and modeling the bladder as an infinite plate, as described above. The bladder tissue may be assumed to behave as a Voigt material so that the shear modulus of elasticity is *µ* = *µ*₁ *+ iωµ*₂, where *µ*₁ and *µ*₂ are the tissue elasticity and viscosity, respectively. Other rheological models and model-free approaches for characterization of tissue mechanical properties can be used instead of the Voigt model. Tissue can be assumed to be either elastic or viscoelastic.

However, as mentioned above, other rheological models, such as Maxwell, generalized Maxwell, Zener, and the like, can be used instead of the Voigt model. Equation 3 can be fitted to obtain Lamb wave dispersion velocities, shown at process block 510, and in these instances the inputs are the Lamb wave velocity versus frequency and the wall thickness (*h*) estimated from the B-mode scanning of the ultrasound system. In addition, due to the limited spatial resolution of the B-mode images, there is some error in wall thickness (*h*) estimates, which are used to fit the dispersion data with the Lamb wave dispersion equation (2). However, the error in Lamb wave dispersion velocity due to errors in wall thickness (*h*) measurements is not larger than 0.2 m/s, or around 10%. Alternatively, higher frequency ultrasound could be used to improve the accuracy of the wall thickness (*h*) measurements.

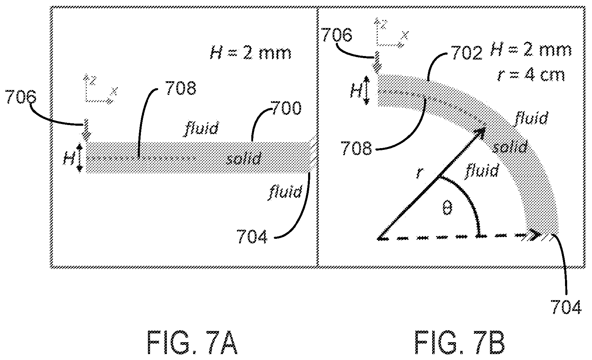

Referring particularly to process block 512 of FIG. 5D, finite element analysis of Lamb wave propagation in a bladder wall, modeled by a flat viscoelastic plate 700 and a curved viscoelastic plate 702, shown in FIGS. 7A and 7B, can be designed to study the effect of curvature on Lamb wave dispersion. Two-dimensional axisymmetric finite element models (FEM) of solid viscoelastic plates 700, 702 can be submerged in an incompressible urine and blood-mimicking fluid, which may be designed using ABAQUS 6.8-3 (SIMULIA, Providence, RI). The fluid surrounding the plates 700, 702 may be represented with acoustic elements (ACAX8) with a bulk modulus, for example, of 2.2 GPa and density, for example, of 1 g/cm³. The equilibrium equation governing the inviscid fluid under the influences of small motion is: $\frac{\partial p}{\partial x} + γ \dot{u} + ρ {u}^{¨} = 0 ,$

Where *p* is the dynamic pressure, *x* is the spatial position, *u̇* and *ü* are the velocity and acceleration of the fluid particles, respectively and *ρ* is the density of the fluid. *γ* is defined as the "volumetric drag" in unit of force per volume per velocity. The constitutive behavior of the fluid that links the dynamic pressure *p*, bulk modulus *K* and volumetric strain *ε* is formulated as: $p = - Kε .$

The acoustic medium and solid parts are coupled with a "Tie" boundary condition and acoustic impedance, for example, at 1.5×10⁶ N·s·m⁻³ is prescribed to the interface.

Both the flat plate 700 and the curved plate 702 may be viscoelastic and have, for example, a density of 1.0 g/cm³, Poisson's ratio of 0.499, Young's modulus of elasticity *E =* 25 kPa and the mechanical properties defined in terms of the two-parameter Prony series where *g*₁ = 0.9901 and *τ*₁ = 2.4x10⁻⁶ s, for example. The Prony series may readily be related to the generalized Maxwell model, which may further be related to the Voigt model so that the chosen mechanical properties estimate a Voigt material where *µ*₁ = 8.33 kPa and *µ*₂ = 2 Pa·s, for example. The plates 700, 702 can be meshed with 8-node biquadratic axisymmetric elements (CAX8H) in ABAQUS.

The flat plate 700 can be, for example, 2 mm thick and 60 mm long. The curved plate 702 can be, for example, 2 mm thick, with 4 cm inner radius, r, and 90 degrees of curvature. In both models shown in FIGS. 7A and 7B, a far end 704 of the plates 700, 702 may have a fixed boundary condition to avoid rigid motion and the plates 700, 702 may be large enough so no reflections occur. An impulsive displacement, implemented as a step function with the amplitude of 100 µm for 500 µs, for example, from a line source through the thickness of the plate, H, can be used to excite the motion in both models. The location of the excitation 706 is noted in FIGS. 7A and 7B. The models can be solved with ABAQUS's explicit dynamic solver, as discussed previously.

The displacement can be measured, for example, every 0.5 mm along the x-axis, as shown in FIGS. 7A and 7B, at a sampling rate of 10 kHz for a total of 20 ms, for example. In the flat plate 700, the displacement can be recorded parallel to the excitation 706, which is shown along the z-axis, at, for example, 11 evenly spaced depths of the sample. In the curved plate 702, the displacement can be recorded at, for example, 11 evenly spaced depths both parallel to the excitation 706, shown along the z-axis, and perpendicular to the arc of the sample, shown along the *r*-axis. The middle depth line 708 can be used for analysis. The Fourier space analysis, shown at process block 508 of FIG. 5D, of the displacement versus time for both plates 700, 702 can be used to obtain Lamb wave dispersion curves. The Lamb wave dispersion equation (2) with the mechanical properties inserted in the FEM simulation can be plotted for comparison. Since the UBV technique measures the axial motion on the curved surface, the motion component parallel to the excitation 706 and linear transducer elements (not shown) can be detected. The comparison between the motion parallel to the excitation and the motion perpendicular to the arc can be carried out in order to determine whether correcting for the curvature in the displacement vector (*dx = rdθ*) is sufficient to account for the curvature of the plate 702.

Referring specifically now to process blocks 508 and 510 of FIG. 5D, the impulse contains frequency components, as previously discussed. A short finite length toneburst generates frequency components up to the inverse of the time duration of the toneburst, which can facilitate extraction of many phase velocities from a single push. The impulse can propagate at a group velocity that is different from the velocities of individual frequency phase velocities and will be discussed below. Because the dispersion diagrams relate to the phase velocities, in some implementations the phase velocities can be extracted at each frequency from the impulse displacement data. As such, a two-dimensional fast Fourier transform (2D FFT) can be performed on the displacement versus time data using (6): ${U}_{Z} \left(k, f\right) = {\sum }_{m = - ∞}^{+ ∞} {\sum }_{n = - ∞}^{+ ∞} {u}_{Z} \left(x, t\right) {e}^{- j 2 π \left(kmx+fnt\right)} ,$
where *u_{z}*(*x,t*) is the motion of the bladder wall perpendicular to the excitation beam 706, as shown in FIGS. 7A and 7B, as a function of distance from the excitation beam (*x*) and time (*t*). Here, *k* is the wave number and *f* is the temporal frequency of the wave. The coordinates of the k-space are the wave number (1/*λ*) and the frequency (*f*). The lowest order mode of propagation carrying the most energy is the zero-order anti-symmetric (A₀) Lamb wave mode. The A₀-mode Lamb wave phase velocity dispersion can be calculated by finding the pixel with the maximum magnitude for each frequency and, since the wave velocity *c =* λ*f,* dividing the frequency coordinate (in Hz) by the wavenumber coordinate (in 1/m) of the k-space. Thus, obtained dispersion data are shown in FIG. 9. The Lamb wave dispersion equation (2) for a flat plate 700 can then be fit to the dispersion data to measure bladder elasticity and viscosity, as well as the estimated thickness of the bladder from the B-mode. In the case of the curved plate 702, the displacement vector may be constructed by recognizing that the arc length *x* = *rθ,* where *r* is the radius of curvature and *θ* the angle. This relationship corrects for the curvature of the curved plate 702.

Alternatively, the group velocity can also be used to assess the properties of the bladder. Although this method may not be as accurate as using the phase velocity to assess properties of the bladder, as discussed previously, the advantage of this method is the simplicity in measurements and calculations. For example, the group velocity approach to assess properties of the bladder does not require a Fourier domain analysis, shown at process block 508 of FIG. 5D, as is used by the previously discussed harmonic excitation and radiation force impulse approaches. Group velocity is calculated by measuring the propagation distance of an impulse as a function of time and distance. The slope of that line is the group velocity. More specifically, one way to measure the group velocity is to measure the time that takes for the peak of the pulse to travel a known distance. This can be done by monitoring the pulse propagation in tissue. The monitoring can be done by correlating the consecutive A-line (radio frequency echo-signals) in a B-mode ultrasound of the region of interest. The time it takes the pulse to travel from its origin (where the push beam was exerted) to a point at a known distance from where the origin is measured. Alternatively, one can measure the time that takes the pulse to pass from one point in the object to another. To obtain more accurate measurements, the process can be repeated at multiple points and the group velocity can be estimated as the slope of the regression line in a distance vs. time plot.

The group velocity is then equal to the phase velocity of the center frequency of the bandwidth and is, therefore, related to the elasticity of the bladder wall. The group velocity (c_{g}) can be related to the elasticity (µ) via the following equation: $μ = ρ * {\left(1.2*{c}_{g}\right)}^{2} ,$
where ρ is the tissue density, assumed to be that of water, for example. A correction factor of 1.2 may be added to correct for the discrepancy between the shear and Lamb wave velocity.

### Non-Limiting Examples

In order to investigate the feasibility of detecting DO through analysis of UBV measurements, the methods described herein were used. GV2 and Pdet data series were gathered from experiments in which neurogenic bladder patients underwent concurrent UBV and UDS measurements. Processing was applied to these data series to identify DO associated transient peaks by decomposing the series into ETS and LE series. Finally, in order to characterize the decomposed series into by a single number, a DO index, *I*, was determined.

### Patient Population

The non-limiting example includes data from 76 adult patients with neurogenic bladder. A urologist's diagnosis from the resulting UDS charts determined that 23 of the patients presented with DO during the UDS measurements.

### UDS and UBV measurements

UBV measurements were collected during the patient's routine cystometry and UDS. During cystometry, saline was infused into the bladder through a catheter instrumented with a pressure sensor. The pressure sustained by the detrusor muscle of the bladder (i.e. Pdet) was inferred by differencing the pressure measurements from the catheter inside the bladder and an additional pressure sensor placed in the patient's vagina or rectum measuring the abdominal pressure. Multiple measurements were obtained at each incremental volume during the filling phase of UDS. At the time of acquisition of the UBV measurement, time stamps were recorded for UBV and UDS measurement and Pdet measurement associated with the UBV measurement was marked.

Multiple UBV measurements (2-3) were collected at each filling volume during cystometry. Histograms of the means and standard deviations were calculated for each experiment of the time intervals between timestamped UBV/UDS measurements. Separate means and standard deviations were collected for measurements taken at the same filling volume and measurement taken between filling volumes.

UBV measurements were collected using a programmable ultrasound system (Verasonics, Redmond, WA), and a curvilinear array transducer (C4-2, ATL/Philips, Bothewell, WA) with a center frequency of 2.5 MHz. A 600-900 microsecond tone burst acoustic pulse was applied to the wall of the bladder to induce waves in the tissue through the mechanism of acoustic radiation force, and an ultrafast plane wave imaging sequence (2500 frames per second) with 3 steering angles for coherent compounding captured the resulting Lamb wave propagation along the bladder wall. Particle motion velocity was estimated from the resulting in-phase and quadrature (IQ) data using an autocorrelation technique. The group velocity (GV) of the Lamb wave propagating along the wall of the bladder was then estimated. Because elasticity is expected to correlate with the square of the wavespeed, as is more rigorously the case with bulk waves, the group velocity was squared (GV2) for analysis.

### Data Preparation and Peak Identification

In order to characterize fluctuations in GV2 and Pdet measurement series associated with DO, such fluctuations were separated from any larger overall trend associated with bladder filling. Fluctuations that were not likely to be a result of bladder wall activity and that can dominate the characterization, such as large outlier measurements were removed. For the latter consideration, a statistical filtering approach (e.g. Hampel filtering) was used. To separate DO associated fluctuations from the main trend of the series, the series was decomposed into LE and ETP series respectively.

Prior to peak identification, values of GV2 and Pdet in which a bladder leak was reported were removed from the given data series. In this example, measurements at the final volume of the experiment were also excluded from the peak identification process in order to avoid the inclusion of normal detrusor contraction associated with the onset of voiding. Such outliers could be due to, for instance, patient motion during measurement or unusually weak UBV signals. In order to remove these outliers, a statistical outlier filter called a Hampel filter was applied to the GV2 and Pdet data series. This filter works by replacing points determined to be outliers with the median of the sample window. The filter uses the following criteria for identifying outliers: A given sample from the data series, xᵢ, which is the center point of sliding window with a median of mᵢ, was considered an outlier if $\left|{x}_{i}-{m}_{i}\right| > {n}_{σ} {σ}_{i}$

Here, n_{σ} is a threshold number of standard deviations and σᵢ was the estimated standard deviation of the sampling window. The standard deviation of the sampling window was estimated from the median absolute deviation as ${σ}_{i} = kmedian \left(\left|{x}_{i - w}-{m}_{i}\right|,…,\left|{x}_{i + w}-{m}_{i}\right|\right)$

Where w is the number of pixels on either side of the centerpoint of the window and the parameter, k, scales the median absolute deviation to the standard deviation. For a standard normal distribution k ≈ 1.48340,41. For analysis of the UBV data, n_{σ} was set to 6 and w was set to 5 for the following reasons. Although the filter does not permit a fixed time window for unevenly sampled data, it does not modify the signal except where an outlier was detected. Likewise the window was set to be large relative to the length of the signal (11 points within the window for w = 5 versus ~16 points based on the median sampling interval and median duration). By setting the outlier threshold conservatively in terms of the number of anomalous outliers it is likely to identify, the filter can be configured to only alter extreme outlying points in the series and to otherwise leave the remaining points unaltered.

Because few points were modified by the filter and the sample window was large, the effect of unfixed time window was expected to be minimal. Setting n_{σ} to 6 means that, for a Gaussian distribution, 99.9997% of the data is left unaltered. It is contemplated that a filter window with a Gaussian distribution will provide a reliable and objective heuristic for identifying anomalous outliers within the data series, and which does not rely on human judgment.

After outlier filtering, the resulting data series were decomposed into ETP and LE series. A smoothing filter was applied to the raw data series (x) to capture any trend in the data series associated with bladder filling. Smoothing was applied to the series through a 2^{nd}-order Savitzky-Golay filter. The Savitzky-Golay filter applies a (2^{nd} order) polynomial regression to data within a sliding window. Because the time intervals were irregularly spaced, a smoothing factor was used in place of a discrete window size. The smoothing factor takes on a value between 0 and 1, which corresponds to the percent of signal energy being attenuated by smoothing (e.g. a smoothing factor of 0.5 would attenuate 50% of the signal energy). The smoothing factor was set to 0.95 in order to filter all fluctuations in the data series except for the main trend of the data.

After smoothing, x was differenced with the filtered data series (xₛ) and time-points associated with negative values of the differenced series are identified (i.e. time points were (x-xₛ)<0).The start and end time-points were also included in the identified time points, so that increases at the time boundary are not treated as transient peaks.

Values from x at the identified time-points were then collected. Linear interpolation was then used to assign values at the time-points that were not identified. The resulting series had the same number of elements as x. We refer to the resulting series as the LE series (x_{LE}).

The ETP series (x_{ETP}) was then determined by differencing the x_{LE} from x. The ETP and LE series can then be used for further analysis.

### Diagnostic Metric

In order to make comparisons between DO and non-DO bladders relative to the peaks identified in the previous section, a single metric that characterizes the identified transient peaks was used. Additionally, such metric should be, in principle, comparable from experiment to experiment given the variable experimental durations and ranges of UBV measurements previously noted. This metric, called DO index, is denoted by *I*: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}}$

Here x_{ETP}(t) denotes the continuous signal of the ETP data series and x_{LE}(t) the continuous signal of the LE data series, each as a function of time, for a given experiment. Essentially, *I* is the square root of the ratio of signal energies (denoted as E_{ETP} and E_{LE} respectively) from the transient peaks and the lower envelope. As such, it is expected that data series from bladders with DO will have a larger signal energy in x_{ETP}(t) relative to x_{LE}(t), and thus a larger *I* than bladders without DO. Because *I* is unitless, it is reasonable to make comparisons between experiments as longer experimental durations and wider ranges in measured GV2 or Pdet are accounted for by normalizing RMS(x_{ETP}) by RMS(x_{LE}).

ROC analysis showed this analysis produced a sensitivity of 0.70 and a specificity of 0.75 for DO. This is comparable to the sensitivity and specificity produced by applying the same analysis to the concurrent Pdet measurements recorded from UDS (0.70 and 0.83, respectively).

The UBV measurements were conducted concurrently with UDS measurements at increasing filling volumes. The advantage of this approach is that the UBV and concurrent UDS measurements were collected at the same time as the UDS charts used for urologist diagnosis of DO (ground truth). This approach would remove the variability that would be introduced if the clinical examination and the UBV study were performed at different times. Another benefit of this approach is that it is possible to compare the peak analysis to both GV2 and Pdet that were acquired at the same time. Applying the methods to Pdet from UDS data and GV2 from UBV data provide similar diagnostic performance. UBV and UDS provided similar information relative to the proposed diagnostic index. UBV thus provides a noninvasive alternative to UDS for DO diagnosis.

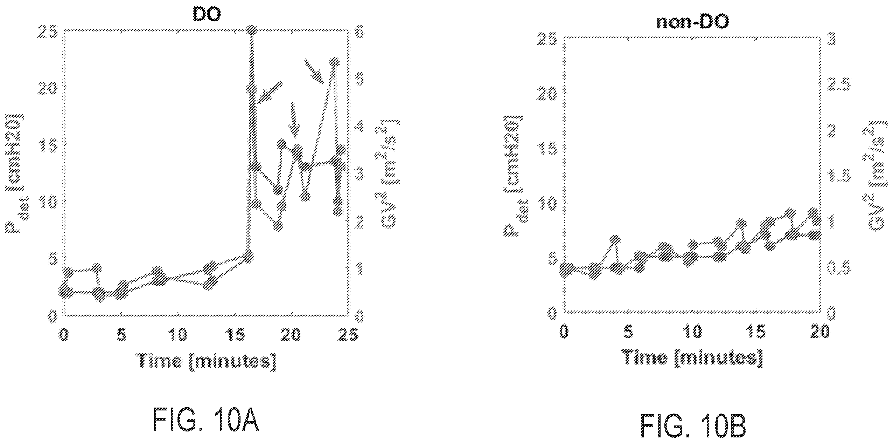

Referring to FIGS. 10A-B, the validation of the above described UBV method for measuring bladder activity is shown. Non-limiting example GV2 and Pdet data series for DO (FIG. 10A) and non-DO (FIG. 10B) bladders. FIGS. 10A-B depict dual-axis plots of both data series, Pdet and GV2. The arrows in FIG. 10A indicate the peaks associated with DO.

These peaks may be separable from the larger trend of the data series when decomposing the series into energies from the transient peaks (ETP) and lower envelope (LE) signals respectively. Although transient fluctuations were observable in the non-DO bladder, the signal energy associated with these fluctuations was comparatively low. This pattern was captured in the DO index, *I*, which is the square root of the ratio of signal ETP and the LE, respectively. In particular, a higher value of *I* was observed in this example for both Pdet and GV2 in the example bladder with DO versus that of the bladder without DO.

Statistical and diagnostic analysis of this characterization approach was applied using a non-parametric hypothesis test (Wilcoxon Test) and receiver operator characteristic (ROC) analysis. The resulting analysis is summarized in Table 1. In agreement with the example bladder, median DO indices for GV2 and Pdet were larger in bladders with DO (0.43 and 0.70 respectively) than they were in bladders without DO (0.25 and 0.31). The Wilcoxon test suggests statistical significant stochastic differences in both GV2 and Pdet (p<0.01), implying a statistically significant area under ROC curve (AUC).

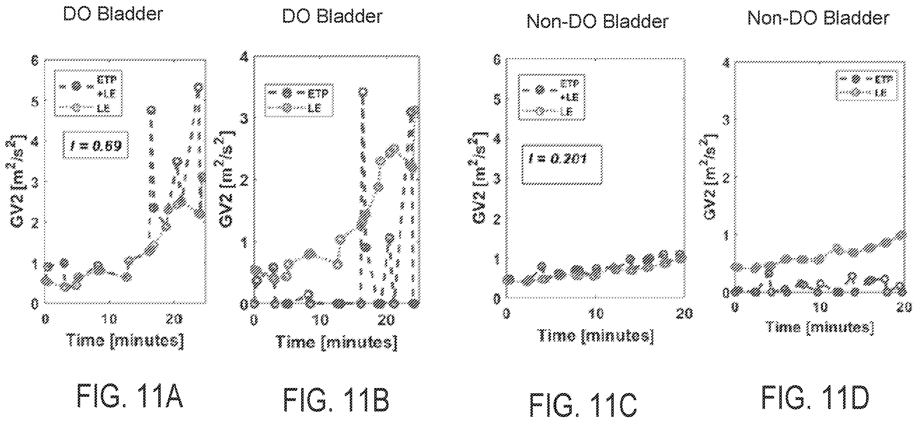

Referring to FIGS. 11A-D, the non-limiting example of FIGS. 10A-B is shown depicting GV2 values with lower envelope (LE) overlaid on the raw series to illustrate the decomposition of the raw series into ETP and LE series. The separated ETP and LE series were used to calculate the signal index (*I*). The identified ensemble of peaks for the data series is the difference between the raw series and the lower envelope.

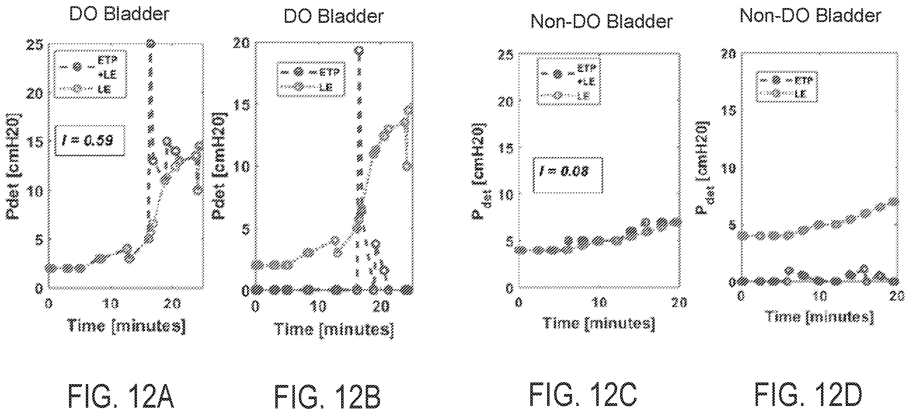

Referring to FIGS. 12A-D, the non-limiting example of FIGS. 10A-B and 11A-D is shown depicting Pdet values with lower envelope (LE) overlaid on the raw series to illustrate the decomposition of the raw series into ETP and LE series. The separated ETP and LE series were used to calculate the signal index (*I*). The identified ensemble of peaks for the data series is the difference between the raw series and the lower envelope.

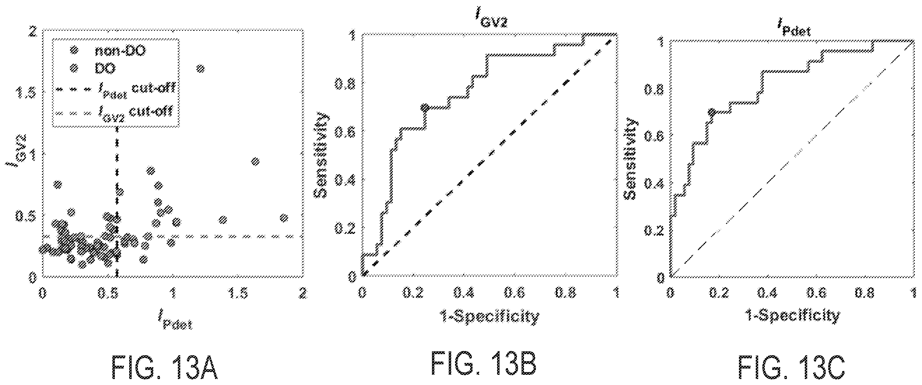

Referring to FIG. 13A-C, non-limiting example graphical results of statistical and diagnostic analyses are shown with the associated ROC curves of *I* calculated for GV2 and Pdet respectively. FIG. 13A is a scatter plot of GV2 signal index against Pdet signal index. The dashed lines denote GV2 and Pdet cut-offs respectively. ROC curves are shown in FIGS. 13B and 13C produced by using the signal index (*I*) as a classifier for DO applied to GV2 (FIG. 13B) and Pdet (FIG. 13C). The circular markers denote the selected optimal cut-off. Optimal cut-offs were estimated using closest-to-(0,1) criterion (i.e., the point on the curve nearest to the upper left corner of the ROC curve). The corresponding cut-off values were 0.327 for I calculated for GV2 and 0.567 for I calculated from Pdet. Their associated sensitivities and specificities are reported in Table 1. FIG. 13A graphically presents these statistical results through a scatter plot of DO indices calculated for UBV/UDS experiments from all 76 patients as well as accompanied ROC curves for GV2 and Pdet (FIG. 13B, FIG. 13C)). Classification characteristics of peak characterization were comparable between GV2 and Pdet (i.e., comparable sensitivity, specificity, and AUC).

**Table 1**

| | GV2 | Pdet |
|---|---|---|
| Median *I* (DO) | 0.43 (0.28, 0.51) | 0.70 (0.47, 1.01) |
| Median I (non-DO) | 0.25 (0.20, 0.32) | 0.31 (0.17, 0.52) |
| AUC | 0.76 | 0.81 |
| p-value (Wilcoxon Test) | p<0.01 | p<0.01 |
| Optimal Cut-off | >0.327 | >0.567 |
| Sensitivity | 0.70 | 0.70 |
| Specificity | 0.75 | 0.83 |
| Accuracy | 0.72 | 0.78 |

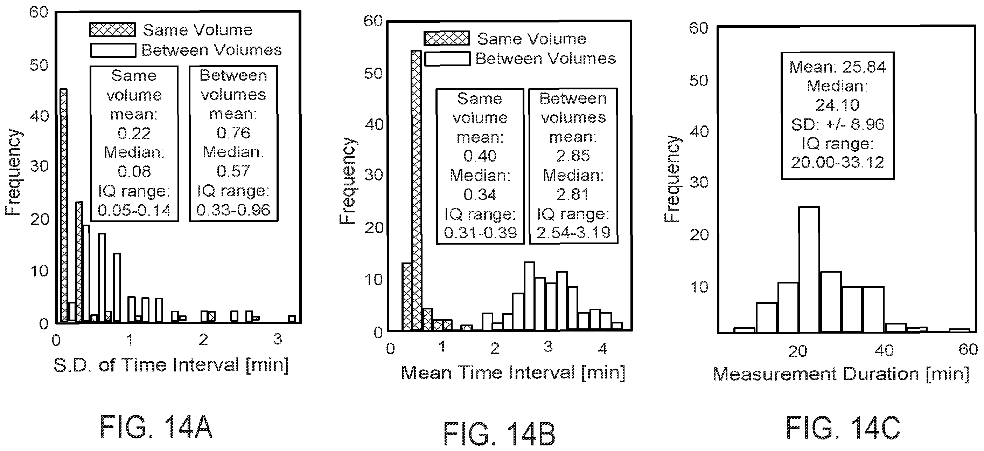

Referring to FIGS. 14A-C, non-limiting example histograms of sampling interval statistic (FIG. 14A and FIG. 14B) and experimental duration (FIG. 14C) for each non-limiting bladder experiment. The standard deviations (SD) (FIG. 14A) and means (FIG. 14B) of sampling intervals of each experiment were calculated for intervals during the same filling volume and intervals between filling volumes. The statistics reported in the text box are for the respective histogram with the interquartile (IQ) range.

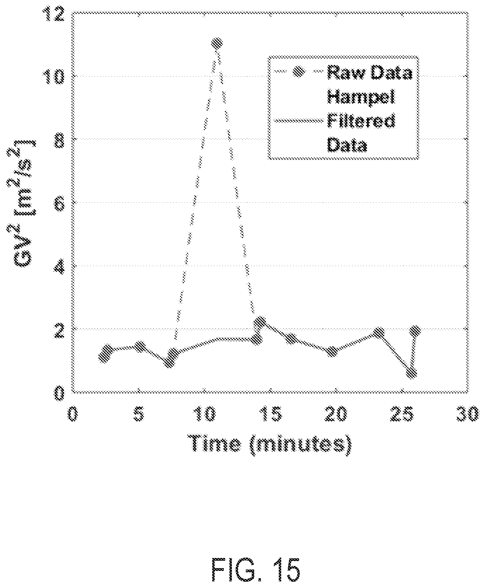

Referring to FIG. 15, a non-limiting example data series before Hampel filtering and after Hampel filtering is shown.

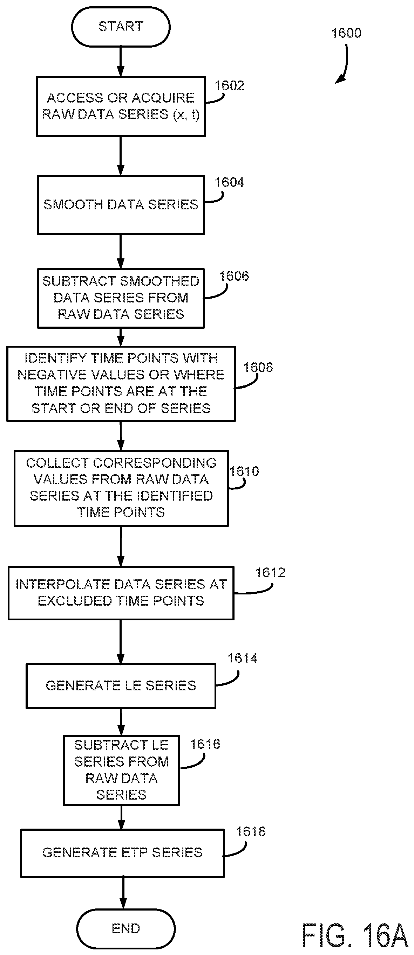

Referring to FIG. 16A, a flow chart of a non-limiting example method 1600 for generating a ETP data series is shown. A raw data series may be accessed or acquired at step 1602, which may be generally in a format of (x, t). Data may be accessed from a data archive system or database, or data may be acquired using a system such as that depicted in FIGS. 1-4. The data series may be smoothed at step 1604. The raw data series may be smoothed using a 2^{nd}-order Savitzky-Golay filter, in a non-limiting example. The smoothed data series may be subtracted from the raw data series at step 1606. The time points with negative values or where time points are at the start or end of the resulting series may be identified at step 1608. Corresponding values from raw data series "x" at the identified time points may be collected at step 1610. The raw data series may be interpolated at the excluded time points at step 1612. A lower envelope (LE) data series may be generated at step 1614 as described above. The LE data series may be subtracted from the raw data series at step 1616 and an ETP data series may be generated at step 1618.

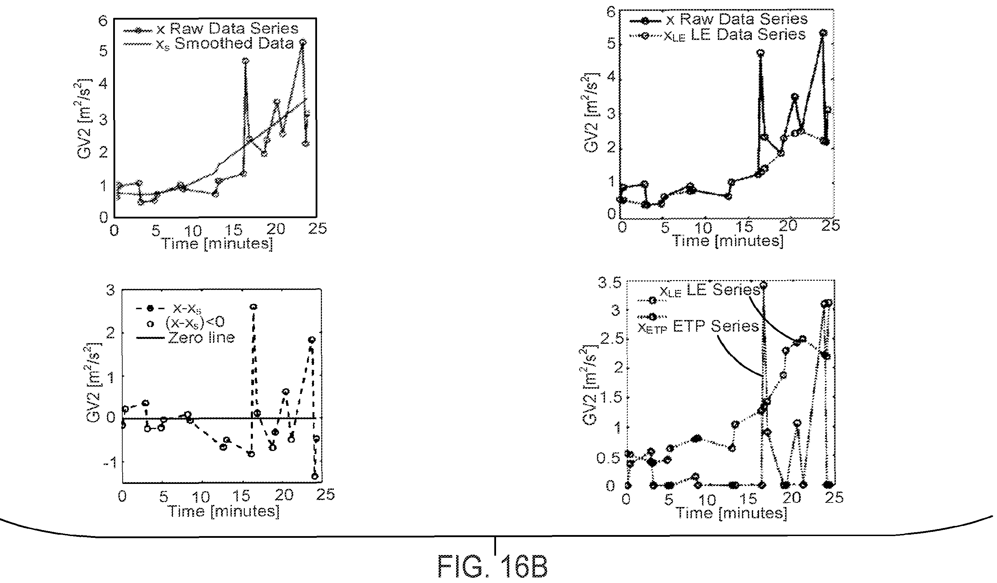

Referring to FIG. 16B, non-limiting example graphs are shown of a raw data series and after processing. A raw data series is shown smoothed using a 2^{nd}-order Savitzky-Golay filter. The Raw data was differenced by the smoothed data and the time-points where values falling below the smoothed curve (i.e. having a negative value) were identified. The identified time points were used to define the LE series from the raw data series by interpolating points. The difference between the LE series and the raw data series determined the ETP series.

It is understood that a method and system for evaluating a parameter representing a property of a tissue volume may require the use of a data-processing electronic circuitry (a processor) controlled by instructions stored in a tangible, non-transient memory. The memory may be random access memory (RAM), read-only memory (ROM), flash memory or any other memory, or combination thereof, suitable for storing control software or other instructions and data. Those skilled in the art should also readily appreciate that instructions or programs defining the functions of the methods described in the present disclosure (such as collection and/or storing of experimental data; data processing to define the sought-after parameters, for example) may be delivered to a processor in many forms, including, but not limited to, information permanently stored on non-writable storage media (e.g. read-only memory devices within a computer, such as ROM, or devices readable by a computer I/O attachment, such as CD-ROM or DVD disks), information alterably stored on writable storage media (e.g. floppy disks, removable flash memory and hard drives) or information conveyed to a computer through communication media, including wired or wireless computer networks. In addition, while the methods described in the present disclosure may be embodied in software, the functions used to implement the methods may optionally or alternatively be embodied in part or in whole using firmware and/or hardware components, such as combinatorial logic, Application Specific Integrated Circuits (ASICs), Field-Programmable Gate Arrays (FPGAs) or other hardware or some combination of hardware, software and/or firmware components. Accordingly, systems and methods employing a processor specifically programmed to perform the steps described in the present disclosure and computer program products containing the set of instructions governing the processor to perform such steps are within the scope of the disclosure.

The present invention has been described in terms of one or more preferred embodiments, and it should be appreciated that many alternatives, variations, and modifications, aside from those expressly stated, are possible and within the scope of the invention as defined by the appended claims. For example, although the methods described in the present disclosure are based on spectral analysis of the ultrasound echo data, it is appreciated that a time-domain analysis can be alternatively employed to determine the dispersion data. In one example, the group velocity or the velocity of the wave peak can be correlated with bladder muscle pressure. Similarly, the complex propagation of a mechanical wave in a soft tissue can be approximated with a shear-wave model (based on consideration of a wave for which the displacement of object particles is perpendicular to the direction of wave propagation, and by definition there are no boundaries in the medium that would affect such propagation) or with a wave model based on the Lamb wave (which is defined for the wave propagates along a plate, or parallel to the surface, with particles moving perpendicularly to the plate surface and, therefore, perpendicularly to the wave propagation direction). In the Lamb-wave based model, the two surfaces of the (infinite) plate are the boundaries that guide the wave forward. In the case of waves propagating on the bladder wall, as in some embodiments described in the present disclosure, a Lamb wave model provides a practical and good approximation. However, other wave models (such as shear wave model referred to above) may also be generally used to approximate the wave motion on the bladder.

## Claims

1. A method for characterizing a parameter representing a property of a tissue volume, the method comprising:
with a transducer (1014), detecting ultrasonic energy reflected by multiple locations along the tissue volume (1006) that is subject to stress to form ultrasonic echo data, wherein the tissue volume is formed by a tissue wall that spatially separates a fluid material from a rigid material;
determining a time series of squared group velocities from the ultrasonic echo data;
forming a lower envelope data series from the time series of squared group velocities based on a difference between the time series of squared group velocities and smoothed data generated by smoothing the time series of squared group velocities with a smoothing filter;
generating an ensemble of transient peaks from the determined time series of squared group velocities by subtracting the lower envelope data series from the time series of squared group velocities; and
determining the parameter representing a property of the tissue volume from the ensemble of transient peaks.

2. The method according to claim 1, wherein the parameter representing a property of a tissue volume is a detrusor overactivity index of the tissue volume.

3. The method according to claim 1, further comprising removing outlier data points from the time series of squared group velocities data and interpolating the time series of squared group velocities data over the removed outlier data points.

4. The method according to claim 1, wherein detecting the ultrasonic energy with the transducer includes causing the stress by applying to the tissue volume at least one of (i) an acoustic radiation force, (ii) an electro-mechanical input; and (iii) a mechanical input.

5. The method according to claim 1, wherein the tissue volume includes a urinary bladder of a subject.

6. The method according to claim 2, wherein the detrusor overactivity (DO) index *I* is determined by: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$ where x_{ETP}(t) represents a continuous signal of the ensemble of transient peaks (ETP) data series and x_{LE}(t) represents a continuous signal of the lower envelope (LE) data series, each as a function of time.

7. A system for characterizing a parameter representing a property of a tissue volume, the system comprising:
a transducer (1014) configured for detecting ultrasonic energy reflected by multiple locations along the tissue volume (1006) that is subject to stress to form ultrasonic echo data, wherein the tissue volume is formed by a tissue wall that spatially separates a fluid material from a rigid material;
a processor (102) configured to:
determine a time series of squared group velocities from the ultrasonic echo data;
form a lower envelope data series from the time series of squared group velocities based on a difference between the time series of squared group velocities and smoothed data generated by smoothing the time series of squared group velocities with a smoothing filter;
generate an ensemble of transient peaks from the determined time series of squared group velocities by subtracting the lower envelope data series from the time series of squared group velocities; and
determine the parameter representing a property of the tissue volume from the ensemble of transient peaks.

8. The system according to claim 7, wherein the parameter representing a property of the tissue volume is a detrusor overactivity index of the tissue volume.

9. The system according to claim 7, wherein the processor is further configured to remove outlier data points from the time series of squared group velocities data and interpolate the wave speed time series data over the removed outlier data points.

10. The system according to claim 7, wherein the transducer is configured to detect ultrasonic energy by causing the stress by applying to the tissue volume at least one of (i) an acoustic radiation force, (ii) an electro-mechanical input; and (iii) a mechanical input.

11. The system according to claim 7, wherein the tissue volume includes a urinary bladder of a subject.

12. The system according to claim 8, wherein the detrusor overactivity (DO) index *I* is determined by: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$ where x_{ETP}(t) represents a continuous signal of the ensemble of transient peaks (ETP) data series and x_{LE}(t) represents a continuous signal of the lower envelope (LE) data series, each as a function of time.

## Patentansprüche

1. Verfahren zum Charakterisieren eines Parameters, der eine Eigenschaft eines Gewebevolumens repräsentiert, wobei das Verfahren umfasst:
mit einem Wandler (1014) Ultraschallenergie zu erfassen, die von mehreren Stellen entlang des Gewebevolumens (1006), das einer Belastung ausgesetzt ist, reflektiert wird, um Ultraschallechodaten zu bilden, wobei das Gewebevolumen durch eine Gewebewand gebildet wird, die ein flüssiges Material räumlich von einem starren Material trennt;
aus den Ultraschallechodaten einer Zeitreihe von quadrierten Gruppengeschwindigkeiten zu bestimmen;
aus der Zeitreihe der quadrierten Gruppengeschwindigkeiten eine untere Hüllkurven-Datenreihe zu bilden auf der Grundlage einer Differenz zwischen der Zeitreihe der quadrierten Gruppengeschwindigkeiten und geglätteten Daten, die durch Glätten der Zeitreihe der quadrierten Gruppengeschwindigkeiten mit einem Glättungsfilter erzeugt wurden;
aus der ermittelten Zeitreihe der quadrierten Gruppengeschwindigkeiten durch Subtrahieren der unteren Hüllkurven-Datenreihe von der Zeitreihe der quadrierten Gruppengeschwindigkeiten ein Ensemble von transienten Spitzen zu erzeugen; und
den Parameter, der eine Eigenschaft des Gewebevolumens repräsentiert, aus dem Ensemble von transienten Spitzen zu bestimmen.

2. Verfahren nach Anspruch 1, wobei der Parameter, der eine Eigenschaft eines Gewebevolumens repräsentiert, ein Detrusor-Hyperaktivitätsindex des Gewebevolumens ist.

3. Verfahren nach Anspruch 1, das ferner das Entfernen von Ausreißer-Datenpunkten aus der Zeitreihe der quadrierten Gruppengeschwindigkeitsdaten und das Interpolieren der Zeitreihe der quadrierten Gruppengeschwindigkeitsdaten über die entfernten Ausreißer-Datenpunkte umfasst.

4. Verfahren nach Anspruch 1, wobei das Erfassen der Ultraschallenergie mit dem Wandler das Erzeugen der Belastung durch Anlegen mindestens einer der folgenden Kräfte an das Gewebevolumen umfasst: (i) eine akustische Strahlungskraft, (ii) eine elektromechanische Eingangsgröße und (iii) eine mechanische Eingangsgröße.

5. Verfahren nach Anspruch 1, wobei das Gewebevolumen eine Harnblase eines Probanden umfasst.

6. Verfahren nach Anspruch 2, wobei der Detrusor-Hyperaktivität (DO)-Index I bestimmt wird durch: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$ wobei x_{ETP}(t) ein kontinuierliches Signal der Datenreihe der transienten Spitzen (ETP) darstellt und x_{LE}(t) ein kontinuierliches Signal der Datenreihe der unteren Hüllkurve (LE) darstellt, jeweils als Funktion der Zeit.

7. System zum Charakterisieren eines Parameters, der eine Eigenschaft eines Gewebevolumens repräsentiert, wobei das System umfasst:
einen Wandler (1014), der dafür eingerichtet ist, Ultraschallenergie zu erfassen, die von mehreren Stellen entlang des Gewebevolumens (1006), das einer Belastung ausgesetzt ist, reflektiert wird, um Ultraschallechodaten zu erzeugen, wobei das Gewebevolumen durch eine Gewebewand gebildet wird, die ein flüssiges Material räumlich von einem starren Material trennt;
einen Prozessor (102), der dafür eingerichtet ist,
aus den Ultraschallechodaten eine Zeitreihe von quadrierten Gruppengeschwindigkeiten zu bestimmen;
aus der Zeitreihe der quadrierten Gruppengeschwindigkeiten eine untere Hüllkurven-Datenreihe zu bilden auf der Grundlage einer Differenz zwischen der Zeitreihe der quadrierten Gruppengeschwindigkeiten und geglätteten Daten, die durch Glätten der Zeitreihe der quadrierten Gruppengeschwindigkeiten mit einem Glättungsfilter erzeugt wurden;
aus der ermittelten Zeitreihe der quadrierten Gruppengeschwindigkeiten durch Subtrahieren der unteren Hüllkurven-Datenreihe von der Zeitreihe der quadrierten Gruppengeschwindigkeiten ein Ensemble von transienten Spitzen zu erzeugen; und
den Parameter, der eine Eigenschaft des Gewebevolumens repräsentiert, aus dem Ensemble von transienten Spitzen zu bestimmen.

8. System nach Anspruch 7, wobei der Parameter, der eine Eigenschaft des Gewebevolumens darstellt, ein Detrusor-Hyperaktivitätsindex des Gewebevolumens ist.

9. System nach Anspruch 7, wobei der Prozessor ferner dafür eingerichtet ist, Ausreißer-Datenpunkte aus der Zeitreihe der quadrierten Gruppengeschwindigkeitsdaten zu entfernen und die Zeitreihendaten der Wellengeschwindigkeit über die entfernten Ausreißer-Datenpunkte zu interpolieren.

10. System nach Anspruch 7, wobei der Wandler dafür eingerichtet ist, Ultraschallenergie zu erfassen, indem die Belastung durch Anlegen mindestens einer der folgenden Kräfte an das Gewebevolumen erzeugt wird: (i) eine akustische Strahlungskraft, (ii) eine elektromechanische Eingangsgröße und (iii) eine mechanische Eingangsgröße.

11. System nach Anspruch 7, wobei das Gewebevolumen eine Harnblase eines Probanden umfasst.

12. Das System nach Anspruch 8, wobei der Detrusor-Hyperaktivität (DO)-Index *I* bestimmt wird durch: $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$ wobei x_{ETP} (t) ein kontinuierliches Signal der Datenreihe der transienten Spitzen (ETP) darstellt und x_{LE} (t) ein kontinuierliches Signal der Datenreihe der unteren Hüllkurve (LE) darstellt, jeweils als Funktion der Zeit.

## Revendications

1. Méthode pour caractériser un paramètre représentant une propriété d'un volume de tissu, la méthode comprenant :
avec un transducteur (1014), la détection d'énergie ultrasonore réfléchie par de multiples emplacements le long du volume de tissu (1006) qui est soumis à une contrainte pour former des données d'écho ultrasonore, dans laquelle le volume de tissu est formé par une paroi de tissu qui sépare spatialement un matériau fluide d'un matériau rigide ;
la détermination d'une série temporelle de vitesses de groupe au carré à partir des données d'écho ultrasonore ;
la formation d'une série de données d'enveloppe inférieure à partir de la série temporelle de vitesses de groupe au carré sur la base d'une différence entre la série temporelle de vitesses de groupe au carré et les données lissées générées par le lissage de la série temporelle de vitesses de groupe au carré avec un filtre de lissage ;
la génération d'un ensemble de pics transitoires à partir de la série temporelle déterminée de vitesses de groupe au carré en soustrayant la série de données d'enveloppe inférieure de la série temporelle de vitesses de groupe au carré ; et
la détermination du paramètre représentant une propriété du volume de tissu à partir de l'ensemble de pics transitoires.

2. Méthode selon la revendication 1, dans laquelle le paramètre représentant une propriété d'un volume de tissu est un indice d'hyperactivité du détrusor du volume de tissu.

3. Méthode selon la revendication 1, comprenant en outre la suppression des points de données aberrants de la série temporelle de données de vitesses de groupe au carré et l'interpolation de la série temporelle de données de vitesses de groupe au carré sur les points de données aberrants supprimés.

4. Méthode selon la revendication 1, dans laquelle la détection de l'énergie ultrasonore avec le transducteur inclut la provocation de la contrainte en appliquant au volume de tissu au moins l'un parmi (i) une force de radiation acoustique, (ii) une entrée électromécanique ; et (iii) une entrée mécanique.

5. Méthode selon la revendication 1, dans laquelle le volume de tissu inclut une vessie urinaire d'un sujet.

6. Méthode selon la revendication 2, dans laquelle l'indice d'hyperactivité du détrusor (DO) *I* est déterminé par : $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$ où x_{ETP}(t) représente un signal continu de l'ensemble de séries de données de pics transitoires (ETP) et x_{LE}(t) représente un signal continu des séries de données d'enveloppe inférieure (LE), chacun en fonction du temps.

7. Système pour caractériser un paramètre représentant une propriété d'un volume de tissu, le système comprenant :
un transducteur (1014) configuré pour détecter une énergie ultrasonore réfléchie par de multiples emplacements le long du volume de tissu (1006) qui est soumis à une contrainte pour former des données d'écho ultrasonore, dans lequel le volume de tissu est formé par une paroi de tissu qui sépare spatialement un matériau fluide d'un matériau rigide ;
un processeur (102) configuré pour :
déterminer une série temporelle de vitesses de groupe au carré à partir des données d'écho ultrasonore ;
former une série de données d'enveloppe inférieure à partir de la série temporelle de vitesses de groupe au carré sur la base d'une différence entre la série temporelle de vitesses de groupe au carré et les données lissées générées par le lissage de la série temporelle de vitesses de groupe au carré avec un filtre de lissage ;
générer un ensemble de pics transitoires à partir de la série temporelle déterminée de vitesses de groupe au carré en soustrayant la série de données d'enveloppe inférieure de la série temporelle de vitesses de groupe au carré ; et
déterminer le paramètre représentant une propriété du volume de tissu à partir de l'ensemble de pics transitoires.

8. Système selon la revendication 7, dans lequel le paramètre représentant une propriété du volume de tissu est un indice d'hyperactivité du détrusor du volume de tissu.

9. Système selon la revendication 7, dans lequel le processeur est en outre configuré pour supprimer les points de données aberrants de la série temporelle de données de vitesses de groupe au carré et interpoler les données de série temporelle de vitesse des ondes sur les points de données aberrants supprimés.

10. Système selon la revendication 7, dans lequel le transducteur est configuré pour détecter de l'énergie ultrasonore en provoquant la contrainte en appliquant au volume de tissu au moins l'un parmi (i) une force de radiation acoustique, (ii) une entrée électromécanique ; et (iii) une entrée mécanique.

11. Système selon la revendication 7, dans lequel le volume de tissu inclut une vessie urinaire d'un sujet.

12. Système selon la revendication 8, dans lequel l'indice d'hyperactivité du détrusor (DO) *I* est déterminé par : $I = \frac{RMS \left({x}_{ETP}\right)}{RMS \left({x}_{LE}\right)} \approx \frac{\sqrt{\frac{1}{{T}_{2} - {T}_{1}} {\int }_{{T}_{1}}^{{T}_{2}} {x}_{ETP} {\left(t\right)}^{2}}}{\sqrt{\frac{1}{{T}_{2} - {T}_{1}} {\int }_{{T}_{1}}^{{T}_{2}} {x}_{LE} {\left(t\right)}^{2}}} = \frac{\sqrt{{E}_{ETP}}}{\sqrt{{E}_{LE}}} ;$
où x_{ETP}(t) représente un signal continu de l'ensemble de séries de données de pics transitoires (ETP) et x_{LE}(t) représente un signal continu des séries de données d'enveloppe inférieure (LE), chacun en fonction du temps.
